# EUROPÄISCHE PATENTSCHRIFT

(11) **EP 1 900 712 B1**
(45) Veröffentlichungstag und Bekanntmachung des Hinweises auf die Patenterteilung: **15.06.2016**
(21) Anmeldenummer: 07112234.5
(22) Anmeldetag: 11.07.2007
(51) Int. Cl.: C07C 1/20, C07C 11/09, C07C 29/80, C07C 41/42, C07C 43/04

(54) **Verfahren zur Spaltung von Methyl-tertiär-butylether**
Method for the decomposition of methyl tertiary-butyl ether
Procédé de décomposition d'éther méthylique de l'alcool tertiobutylique

(30) Priorität: 29.08.2006 DE 102006040430
(43) Veröffentlichungstag der Anmeldung: 19.03.2008
(73) Patentinhaber: Evonik Degussa GmbH, 45128 Essen (DE)
(72) Erfinder: Winterberg, Markus, 45711, Datteln (DE); Luh, Walter, 45770, Marl (DE); Santiago Fernandez, Silvia, 33009, Oviedo (ES); Nierlich, Franz, 45768, Marl (DE); Houbrechts, Stephan, B-2570, Duffel (BE); Maschmeyer, Dietrich, 45657, Recklinghausen (DE); Zanthoff, Horst-Werner, 45481, Mülheim a.d. Ruhr (DE); Büschken, Wilfried, 45721, Haltern am See (DE)

(56) Entgegenhaltungen:
- WO-A-2004/007412
- DE-A1- 10 238 370
- US-A- 5 171 920
- R. TROTTA, I. MIRACCA: "Case history: synthesis and decomposition of MTBE" CATALYSIS TODAY, Bd. 34, 1997, Seiten 447-455, XP002465780

## Beschreibung

Die vorliegende Erfindung betrifft ein Verfahren zur Spaltung von Methyl-tert.-butylether (MTBE).

Isobuten ist Ausgangsstoff für die Herstellung einer Vielzahl von Produkten, z. B. für die Herstellung von Butylkautschuk, Polyisobutylen, Isobuten-Oligomeren, verzweigten C₅-Aldehyden, C₅-Carbonsäuren, C₅-Alkoholen und C₅-Olefinen. Weiterhin wird es als Alkylierungsmittel, insbesondere zur Synthese von tert.-Butylaromaten, und als Zwischenprodukt für die Erzeugung von Peroxiden eingesetzt. Darüber hinaus kann Isobuten als Vorstufe für die Herstellung von Methacrylsäure und deren Estern verwendet werden.

In technischen Strömen liegt Isobuten häufig zusammen mit gesättigten und ungesättigten C₄-Kohlenwasserstoffen vor. Aus diesen Gemischen kann Isobuten wegen der geringen Siedepunktsdifferenz bzw. des sehr geringen Trennfaktors zwischen Isobuten und 1-Buten durch Destillation nicht wirtschaftlich abgetrennt werden. Daher wird Isobuten aus technischen Kohlenwasserstoffgemischen üblicherweise dadurch gewonnen, dass Isobuten zu einem Derivat umgesetzt wird, das sich leicht vom übrig gebliebenen Kohlenwasserstoffgemisch abtrennen lässt, und dass das isolierte Derivat zu Isobuten und Derivatisierungsmittel zurückgespaltet wird.

Üblicherweise wird Isobuten aus C₄-Schnitten, beispielsweise der C₄-Fraktion eines Steamcrackers, wie folgt, abgetrennt. Nach Entfernung des größten Teils der mehrfach ungesättigten Kohlenwasserstoffe, hauptsächlich Butadien, durch Extraktion(-sdestillation) oder Selektivhydrierung zu linearen Butenen wird das verbleibende Gemisch (Raffinat I oder hydriertes Crack-C₄) mit Alkohol oder Wasser umgesetzt. Bei der Verwendung von Methanol entsteht aus Isobuten Methyl-tert.-butylether (MTBE) und bei Einsatz von Wasser tert.-Butanol (TBA). Nach ihrer Abtrennung können beide Produkte in Umkehrung ihrer Bildung zu Isobuten gespalten werden.

MTBE ist preiswerter als TBA, weil die Umsetzung von isobutenhaltigen Kohlenwasserstoffen mit Methanol leichter ist als mit Wasser und MTBE als Komponente von Ottokraftstoffen in großen Mengen produziert wird. Die Gewinnung von Isobuten aus MTBE ist daher potentiell wirtschaftlicher als die aus TBA, wenn für die Spaltung von MTBE ein ähnlich gutes Verfahren wie für die Spaltung von TBA zur Verfügung stünde.

Die Spaltung von MTBE kann in der Flüssigphase, Gasflüssigphase oder Gasphase in Gegenwart von sauren Katalysatoren durchgeführt werden. Ganz gleich in welcher Phase die Spaltung durchgeführt wird, entstehen, wenn auch im unterschiedlichen Ausmaß, Nebenprodukte. Beispielsweise können sich aus dem bei der Spaltung entstehenden Isobuten durch sauer katalysierte Dimerisierung oder Oligomerisierung unerwünschte C₈- und C₁₂-Komponenten bilden. Bei den unerwünschten C₈-Komponenten handelt es sich hauptsächlich um 2,4,4-Trimethyl-1-penten und 2,4,4-Trimethyl-2-penten. Darüber hinaus kann sich ein Teil des bei der Spaltung entstehenden Methanols zu Dimethylether umsetzen.

Aus wirtschaftlichen Gründen (niedriger Preis; hohe Verfügbarkeit) wird für die Herstellung von Isobuten durch Spaltung von MTBE, kein hochreines MTBE, wie beispielsweise MTBE/S der Oxeno Olefinchemie GmbH, sondern MTBE in üblicher technischer Qualität (Kraftstoffqualität) verwendet. Technischer MTBE enthält als Nebenkomponenten meistens C₈-Olefine, beispielsweise die oben genannten, 2-Methoxybutan, entstanden bei der Synthese von MTBE aus linearen Butenen und Methanol, TBA, Methanol und gegebenenfalls C₄- und C₅-Kohlenwasserstoffe. Bei der Herstellung von hochreinem MTBE werden diese Verunreinigungen aus technischem MTBE entfernt. Ein entsprechender Destillationsprozess ist in WO2004/007412A beschrieben. Da bei der Entfernung der im technischen MTBE enthaltenen Verunreinigungen große Nebenströme entstehen, ist die Aufarbeitung von technischem MTBE zu hochreinem MTBE sehr kostspielig. Deswegen ist es nicht wirtschaftlich, Isobuten aus hochreinem MTBE herzustellen.

Es sind verschiedene Verfahren zur Herstellung von Isobuten durch Spaltung von MTBE bekannt.

In EP 0 302 336 wird Isobuten durch Spaltung von MTBE in einer Kolonne, die eine Kombination aus Rührkessel, Destillationskolonne und Extraktionskolonne ist, gewonnen. Der saure Katalysator ist im Sumpfumlauf angeordnet. MTBE wird in den Sumpfumlauf eingespeist. Ein Teil des Sumpfumlaufs wird abgezogen. Das rohe Isobuten wird in der Kolonne mit Wasser extrahiert. Der wässrige, Methanol enthaltende Extrakt wird oberhalb des Sumpfes abgezogen. Als Kopfprodukt wird das Isobuten enthalten. Der Sumpfabzug wird nach Ausschleusung einer Teilmenge zurückgeführt.

In EP 0 068 785 wird ein Verfahren beansprucht, bei dem die Spaltung von MTBE in einem kontinuierlich betriebenen Rührkessel erfolgt. Dabei ist der saure Katalysator im Edukt suspendiert. Aus dem abdestillierenden Reaktionsgemisch wird Isobuten über eine Kolonne als Kopfprodukt abgetrennt. Das Sumpfprodukt wird zum Teil in den Rührkessel zurückgeführt. Der andere Teil wird in einem Zweikolonnensystem in einen MTBE-haltigen Strom, der in den Rührkessel zurückgeführt wird, und Methanol, das als Seitenstrom entnommen wird, getrennt. Es wird nicht dargelegt, wie Nebenkomponenten im eingesetzten MTBE und gebildete Nebenprodukte abgetrennt werden.

In DE 32 10 435 wird MTBE in einer Reaktivdestillationskolonne gespalten. Als Kopfprodukt wird ein Isobutengemisch mit geringen Mengen an Methanol und Spuren von Diisobuten erhalten. Das Sumpfprodukt der Reaktivdestillationskolonne wird in einer Destillationskolonne in einem MTBE-haltigen Strom, der in die Reaktivdestillationskolonne zurückgeführt wird, und in ein Sumpfprodukt, das aus Methanol besteht, getrennt. Es wird nicht aufgezeigt, wie Nebenprodukte abgetrennt werden.

In den Schriften EP 0 633 048, DE 100 20 943 und DE 101 11 549 wird die Herstellung von Isobuten durch Spaltung von MTBE in einer Reaktivdestillationskolonne beansprucht. Die Abtrennung von Nebenkomponenten aus dem Spaltgemisch wird nicht offengelegt.

In DE 102 27 350 und DE 102 27 351 werden Verfahren zur Herstellung von Isobuten durch Spaltung von MTBE in der Gasphase beschrieben. In beiden Verfahren wird das Spaltprodukt nach Kondensation mit Wasser extrahiert. Es wird als Extrakt ein Wasser-Methanolgemisch erhalten, das destillativ in Methanol und Wasser aufgetrennt wird. Das Raffinat besteht aus Isobuten, nicht umgesetzten MTBE und Nebenkomponenten. Dieses Gemisch wird durch Destillation in ein Kopfprodukt, das Isobuten mit geringen Mengen an Dimethylether enthält, und in ein Sumpfprodukt, bestehend aus MTBE und Nebenkomponenten, getrennt. Über Verwendung bzw. Aufarbeitung des anfallenden MTBE-Gemisches werden keine Angaben gemacht.

In US 6,049,020 wird u. a. die Herstellung von Isobuten durch Spaltung von MTBE beschrieben. Aus dem Reaktionsprodukt wird durch Extraktion mit Wasser Methanol entfernt. Das verbleibende Raffinat wird destillativ in ein Kopfprodukt, das Isobuten enthält, und ein Sumpfprodukt, bestehend aus nicht umgesetztem MTBE und Nebenkomponenten, getrennt. Die Aufarbeitung des MTBE-Gemisches wird nicht beschrieben. In US 6,072,095 und US 6,143,936 erfolgt die Aufarbeitung des Spaltproduktes analog. Das anfallende MTBE-Gemisch mit den Nebenkomponenten wird nicht aufgearbeitet, es kann in eine Anlage zur Herstellung von MTBE eingeleitet werden.

Bei der Spaltung von technischem MTBE, das 2-Methoxybutan, enthält, entstehen durch Spaltung von 2-Methoxybutan lineare Butene. Keines der oben genannten Verfahren gibt eine Lösung für die Abtrennung der linearen Butene vom Isobuten bzw. eine Begrenzung des Gehalts an linearen Butenen in der Isobutenfraktion an.

R. Trotta und I. Miracca zeigen in Catalysis Today 34 (1997), S 447 bis 455 (Case history: synthesis and decomposition of MTBE) die Abhängigkeit des Gehaltes an linearen Butenen in Isobuten, das durch Spaltung von MTBE erzeugt worden ist, vom 2-Methoxybutan-Gehalt im Einsatz-MTBE und vom MTBE-Umsatz. Der Zusammenhang ist in einem nicht skalierten Diagramm dargestellt. Je höher der 2-Methoxybutangehalt im Einsatz-MTBE und je höher der MTBE-Umsatz ist, desto höher ist der Gehalt an linearen n-Butenen im Spaltprodukt. Aus einem Einsatz-MTBE mit einem Gehalt an 2-Methoxybutan (MSBE) von über 1000 Massen-ppm lässt sich bei vollständigem MTBE-Umsatz kein Isobuten mit einem Gehalt an linearen Butenen von unter 1000 Massen-ppm herstellen. In diesem Falle muss der MTBE-Umsatz verringert werden. Dies hat zur Folge, dass nach Abtrennung des Isobutens eine größere Menge eines Gemisches anfällt, das hauptsächlich aus Methanol und MTBE besteht und eine höhere Konzentration an 2-Methoxybutan aufweist als das Einsatz-MTBE. Dieses Gemisch darf, um die Isobutenspezifikation einzuhalten, wegen seines höheren 2-Methoxybutangehaltes nicht in den Spaltreaktor eingespeist werden. Selbst die Rückführung dieses Stromes in eine vorgeschaltete MTBE-Anlage, die gerade so viel MTBE erzeugt, wie für die Spaltung benötigt wird, ist nicht möglich, da sich 2-Methoxybutan im Anlagesystem anreichern würde. Es ist daher ein Ausschleusestrom mit MTBE-Verluste unvermeidbar.

Aufgabe der vorliegenden Erfindung war deshalb die Bereitstellung eines Verfahrens zur Spaltung von MTBE, mit dem die Spaltung von 2-Methoxybutan aufweisenden MTBE so durchgeführt werden kann, dass das erhaltene Isobuten weniger als 1000 Massen-ppm an linearen Butenen in Bezug auf die C₄-Olefinfraktion aufweist und ein Ausschleusestrom für die Nebenkomponenten klein gehalten werden kann.

Überraschenderweise wurde nun gefunden, dass mit einem MTBE-Spaltverfahren, welches einen Schritt der destillativen Abtrennung von Schwersiedern, insbesondere von 2-Methoxybutan, und anschließend einen Schritt der MTBE-Spaltung aufweist, so durchgeführt werden kann, dass der Gehalt an linearen Butenen (die durch Spaltung von MSBE erhalten werden) im Spaltprodukt auf kleiner 1000 Massen-ppm in Bezug auf das Isobuten gesenkt werden kann.

Dieser Befund ist insbesondere deshalb überraschend, weil R. Trotta und I Miracca in Catalysis Today 34 (1997), Seite 453 schreiben, dass 2-Methoxybutan und MTBE den gleichen Siedepunkt haben und deshalb nicht voneinander getrennt werden können.

Gegenstand der vorliegenden Erfindung ist deshalb ein Verfahren zur Spaltung von Methyl-tert.-butylether (MTBE) nach Anspruch 1. Das Verfahren gemäß der vorliegenden Erfindung weist folgende Vorteile auf: Das Verfahren ist sehr flexibel hinsichtlich der verschiedenen MTBE-Qualitäten, die unterschiedliche Konzentrationen an Nebenprodukten, wie beispielsweise Diisobuten oder 2-Methoxybutan, aufweisen können. Durch Anpassen der Destillations- und/oder der Spaltbedingungen ist es jeweils möglich, ein Isobuten zu erzeugen, das weniger als 1000 Massen-ppm an linearen Butenen bezogen auf die C₄-Olefinfraktion enthält. Das nach Abtrennung des Isobutens aus dem Spaltprodukt verbleibende Gemisch, das Methanol, MTBE und Nebenprodukte enthält, kann vollständig in eine MTBE-Anlage zurückgeführt werden. Dies gilt selbst dann, wenn diese MTBE-Anlage nur so viel MTBE erzeugt, wie für die Spaltung benötigt wird. Bei allein stehenden Anlagen (stand alone plants), bei denen angeliefertes MTBE eingesetzt wird, ist es möglich, den nach Abtrennung von Isobuten und Methanol verbleibenden Rest des Spaltproduktes in die Destillationskolonne zurückzufahren.

Nachfolgend wird die Erfindung beispielhaft beschrieben.

Das Verfahren zur Spaltung von Methyl-tert.-butylether (MTBE), das zumindest die Schritte
a) destillative Trennung eines MTBE aufweisenden Stroms I in einen MTBE aufweisenden Kopfstrom II und einen Sumpfstrom III, der höher als MTBE siedende Verbindungen aufweist, und
b) Spaltung des im Kopfstrom II enthaltenen MTBE an einem Katalysator unter Erhalt eines Spaltproduktes IV aufweist,
   zeichnet sich dadurch aus,
   dass der Strom I einen Anteil an 2-Methoxybutan (MSBE) von größer 1000 Massen-ppm in Bezug auf MTBE aufweist und dass die destillative Trennung in Schritt a) und/oder die Spaltung in Schritt b) so durchgeführt wird, dass das erhaltene Spaltprodukt IV eine Konzentration von weniger als 1000 Massen-ppm an linearen Butenen bezogen auf die C₄-Olefinfraktion aufweist.

### Verfahrensschritt a)

Die destillative Trennung des MTBE aufweisenden Stroms I in einen MTBE aufweisenden Kopfstrom II und einen Sumpfstrom III, der höher als MTBE siedende Verbindungen aufweist, gemäß Verfahrensschritt a) erfolgt in zumindest einer Kolonne, bevorzugt in genau einer Destillationskolonne. In der Kolonne wird der MTBE aufweisende Strom I vorzugsweise aufdestilliert, wobei Schwersieder, insbesondere 2-Methoxybutan, vom MTBE abgetrennt werden. Die eingesetzte Destillationskolonne weist vorzugsweise von 50 bis 140 theoretische Trennstufen, bevorzugt von 60 bis 120 und ganz besonders bevorzugt von 80 bis 110 auf. Das Rücklaufverhältnis, im Rahmen der vorliegenden Erfindung definiert als der Massenstrom des Rücklaufs geteilt durch den Massenstrom des Destillats, beträgt, in Abhängigkeit von der realisierten Stufenzahl, der Zusammensetzung des eingesetzten MTBEs und der erforderlichen Reinheit, vorzugsweise von 1 bis 20, bevorzugt von 1 bis 10. Der Betriebsdruck kann vorzugsweise von 0,1 bis 2,0 MPa_{(abs)} betragen. Wenn die Spaltung des Kopfproduktes II im Spaltreaktor in der Gasphase bei erhöhtem Druck erfolgt, kann es vorteilhaft sein die Destillation bei höherem Druck durchzuführen, wobei in diesem Fall der Kopfkondensator vorzugsweise als Partialkondensator betrieben wird und das Kopfprodukt (II) dampfförmig abgezogen wird. Beträgt der Reaktionsdruck im Spaltreaktor beispielsweise 0,7 MPa(_{abs}), so sollte der Destillationsdruck vorzugsweise mindestens 0,75 MPa_{(abs)} betragen. Bei Betriebsdrucken von größer 1,3 MPa_{(abs)} kann mit der Kondensationswärme ND-Dampf erzeugt werden, mit dem andere Kolonnen des Verfahrens beheizt werden können. Zur Beheizung der Kolonne kann, je nach gewähltem Betriebsdruck, Dampf oder Wärmeträgeröl eingesetzt werden. Das Sumpfprodukt III kann neben den Schwersiedern, wie z. B. 2-Methoxybutan und Diisobuten, auch MTBE enthalten. Dieses Gemisch kann thermisch verwertet werden, als Einsatzstoff für eine Synthesegasanlage genutzt werden oder direkt oder nach Hydrierung als Treibstoffkomponente verwendet werden.

Der Gehalt an 2-Methoxybutan im Kopfstrom II kann durch Variation der Destillationsbedingungen auf unterschiedliche Werte eingestellt werden. Je nach Spaltungsgrad des MTBE im nachgeschalteten Reaktor R sind unterschiedliche 2-Methoxybutan-Konzentrationen im Kopfstrom II zulässig. Wird ein möglichst vollständiger Umsatz des im Strom II enthaltenen MTBE im Reaktor R angestrebt, sollte der Gehalt an 2-Methoxybutan in Strom II 1000 Massen-ppm nicht übersteigen, um die Isobutenspezifikation von weniger als 1000 Massen-ppm linearer Butene einzuhalten; denn bei vollständigem MTBE-Umsatz entspricht das Verhältnis von 2-Methoxybutan zu MTBE annähernd dem Verhältnis von linearen Butenen zu Isobuten im Spaltprodukt. Dies ist nicht im Sinne der Erfindung. Wird dagegen erfindungsgemäß im Reaktor R ein geringerer MTBE-Umsatz eingestellt, kann die 2-Methoxybutan-Konzentration im Strom II mehr als 1000 Massen-ppm betragen, da MTBE schneller als 2-Methoxybutan gespalten wird.

Das Verhältnis der Spaltgeschwindigkeit des MTBE zu der des 2-Methoxybutans kann neben den Spaltbedingungen auch von dem verwendeten Katalysator abhängig sein. Die zulässigen 2-Methoxykonzentrationen im Destillat II können bei vorgegebenen Katalysator und Umsatz durch einfache Vorversuche ermittelt werden. Erfindungsgemäß ist bei Verwendung von wie unten beschriebenen Katalysatoren, die formal aus Magnesiumoxid, Aluminiumoxid und Siliziumoxid bestehen, bei MTBE-Umsätzen von 85 bis 95 % eine 2-Methoxybutan-Konzentration bis 2500 Massen-ppm, insbesondere eine von 2000 Massen-ppm (jeweils bezogen aufs MTBE) zulässig, ohne die Isobutenspezifikation zu gefährden.

### Verfahrensschritt b)

Die Spaltung des im Strom II vorhandenen MTBE in Isobuten und Methanol kann in Gegenwart von sauren Katalysatoren in der Flüssigphase bzw. Gas/Flüssig-Mischphase oder in der Gasphase durchgeführt werden. Im erfindungsgemäßen Verfahren erfolgt die MTBE-Spaltung vorzugsweise in der Gasphase. Bevorzugt erfolgt die MTBE-Spaltung bei einer Temperatur im Bereich von 200 bis 400 °C, vorzugsweise von 230 bis 350 °C.

Vorzugsweise wird die Spaltung in Schritt b) an einem Katalysator durchgeführt, der eine Aktivität in Bezug auf die MTBE-Spaltung aufweist, die zumindest 1 %, vorzugsweise 5 % und besonders bevorzugt 10 % größer ist als die Aktivität in Bezug auf die 2-Methoxybutan-Spaltung. Die Aktivität des Katalysators kann auf einfache Weise dadurch ermittelt werden, dass ein Gemisch aus MSBE und MTBE unter stationären Bedingungen an dem ausgewählten Katalysator zur Reaktion gebracht wird und anschließend das erhaltene Reaktionsprodukt in Bezug auf nicht umgesetzten MTBE und MSBE analysiert wird.

Vorzugsweise werden im erfindungsgemäßen Verfahren zur Spaltung von MTBE, bevorzugt zur Spaltung von MTBE in der Gasphase Katalysatoren verwendet, die formal aus Magnesiumoxid, Aluminiumoxid und Siliciumoxid bestehen. Solche Katalysatoren werden z. B. in US 5,171,920 in Beispiel 4 und in EP 0 589 557 beschrieben.

Gemäß der Erfindung werden Katalysatoren eingesetzt, die formal Magnesiumoxid, Aluminiumoxid und Siliziumdioxid aufweisen, und die einen Anteil an Magnesiumoxid von 0,5 bis 20 Massen-%, bevorzugt von 5 bis 15 Massen-% und besonders bevorzugt von 10 bis 15 Massen-%, einen Anteil an Aluminiumoxid von 4 bis 30 Massen-%, bevorzugt von 10 bis 20 Massen-% und einen Anteil an Siliziumdioxid von 60 bis 95 Massen-%, bevorzugt von 70 bis 90 Massen-% aufweisen. Es kann vorteilhaft sein, wenn der Katalysator neben dem Magnesiumoxid ein Alkalimetalloxid aufweist. Dieses kann z. B. ausgewählt sein aus Na₂O oder K₂O. Vorzugsweise weist der Katalysator als Alkalimetalloxid Na₂O auf. Der bevorzugt eingesetzte Katalysator weist vorzugsweise eine BET-Oberfläche (volumetrisch bestimmt mit Stickstoff gemäß DIN ISO 9277) von 200 bis 450 m²/g, bevorzugt von 200 bis 350 m²/g auf. Wird der erfindungsgemäße Katalysator als aktive Masse auf einem Träger aufgebracht, so weist nur die aktive Masse eine BET-Oberfläche im genannten Bereich auf. Das Material aus Katalysator und Träger kann dagegen je nach Beschaffenheit des Trägers eine deutlich abweichende BET-Oberfläche, insbesondere eine kleinere BET-Oberfläche aufweisen.

Das Porenvolumen des Katalysators beträgt vorzugsweise von 0,5 bis 1,3 ml/g, bevorzugt von 0,65 bis 1,1 ml/g. Das Porenvolumen wird vorzugsweise bestimmt durch die Cyclohexanmethode. Bei dieser Methode wird die zu prüfende Probe zunächst bei 110 °C bis zur Gewichtskonstanz getrocknet. Anschließend werden ca. 50 ml der auf 0,01 g genau eingewogenen Probe in ein gereinigtes und bis zur Gewichtskonstanz getrocknetes Imprägnierrohr gefüllt, dass an der Unterseite eine Auslauföffnung mit einem Schliffhahn aufweist. Die Auslauföffnung ist mit einem kleinen Plättchen aus Polyethylen abgedeckt, wodurch ein Verstopfen der Auslauföffnung durch die Probe verhindert wird. Nach dem Befüllen des Imprägnierrohres mit der Probe wird das Rohr sorgfältig luftdicht verschlossen. Anschließend wird das Imprägnierrohr mit einer Wasserstrahlpumpe verbunden, der Schliffhahn geöffnet und über den Wasserstrahl ein Vakuum im Imprägnierrohr von 20 mbar eingestellt. Das Vakuum kann an einem parallel angeschlossenen Vakuummeter geprüft werden. Nach 20 min. wird Schliffhahn geschlossen und das evakuierte Imprägnierrohr wird anschließend so mit einer Cyclohexanvorlage, in der ein genau abgemessenes Volumen an Cyclohexan vorgelegt wird, verbunden, dass durch Öffnen des Schliffhahns Cyclohexan aus der Vorlage in das Imprägnierrohr gesaugt wird. Der Schliffhahn bleibt so lange geöffnet, bis die gesamte Probe mit Cyclohexan geflutet ist. Anschließend wird der Schliffhahn wieder verschlossen. Nach 15 min wird das Imprägnierrohr vorsichtig belüftet und das nicht absorbierte Cyclohexan in die Vorlage abgelassen. Im Imprägnierrohr bzw. in der Auslauföffnung oder der Verbindung mit der Cyclohexanvorlage anhaftendes Cyclohexan kann durch einen einzelnen vorsichtigen Druckstoß aus einem Saugball, über die Belüftungsleitung in die Vorlage befördert werden. Das Volumen des in der Vorlage vorhandenen Cyclohexans wird notiert. Das Porenvolumen ergibt sich aus dem absorbierten Cyclohexanvolumen, welches bestimmt wird aus dem Cyclohexanvolumen in der Vorlage vor der Messung minus dem Cyclohexanvolumen in der Vorlage nach der Messung, geteilt durch die Masse der untersuchten Probe.

Der mittlere Porendurchmesser (vorzugsweise bestimmt in Anlehnung an DIN 66133) des Katalysators beträgt vorzugsweise von 5 bis 20 nm, bevorzugt von 8 bis 15 nm. Besonders bevorzugt entfällt mindestens 50 %, vorzugsweise über 70 % des Gesamtporenvolumens (Summe des Porenvolumens der Poren mit einem Porendurchmesser von größer-gleich 3,5 nm bestimmt mit Quecksilberporosimetrie gemäß DIN 66133) des Katalysators auf Poren mit einem Durchmesser von 3,5 bis 50 nm (Mesoporen).

Im erfindungsgemäßen Verfahren werden bevorzugt Katalysatoren eingesetzt, die eine mittlere Korngröße (bestimmt durch Siebanalyse) von 10 µm bis 10 mm, vorzugsweise 0,5 mm bis 10 mm, besonders bevorzugt eine mittlere Korngröße von 1 bis 5 mm aufweisen. Vorzugsweise werden feste Katalysatoren eingesetzt, die eine mittlere Korngröße d₅₀, von 2 bis 4 mm, insbesondere von 3 bis 4 mm.

In dem erfindungsgemäßen Verfahren kann der Katalysator als Formkörper eingesetzt werden. Die Formkörper können jegliche Form einnehmen. Bevorzugt wird der Katalysator als Formköper in Form von Kugeln, Extrudaten oder Tabletten eingesetzt. Die Formkörper weisen vorzugsweise die oben genannten mittleren Korngrößen auf.

Der Katalysator kann auch aufgebracht sein auf einen Träger, wie z. B. einen Metall-, Kunststoff- oder Keramikträger, vorzugsweise auf einem in Bezug auf die Reaktion, bei welcher der Katalysator eingesetzt werden soll, inerten Träger. Insbesondere kann der erfindungsgemäße Katalysator auf einen Metallträger, wie z. B. eine Metallplatte oder eine Metallgewebe aufgebracht sein. Solche mit dem erfindungsgemäßen Katalysator ausgerüstete Träger können z. B. als Einbauten in Reaktoren oder Reaktivdestillationskolonnen verwendet werden. Die Träger können auch Metall-, Glas- oder Keramikkugeln oder Kugeln von anorganischen Oxiden sein. Ist der erfindungsgemäße Katalysator auf einem inerten Träger aufgebracht, so wird die Masse und Zusammensetzung des inerten Trägers bei der Bestimmung der Zusammensetzung des Katalysators nicht berücksichtigt.

Die besonders bevorzugt einzusetzenden, formal Magnesiumoxid, Aluminiumoxid (Al₂O₃) und Siliziumdioxid aufweisenden Katalysatoren können z. B. mit einem Verfahren hergestellt werden, das die Schritte
A1) Behandeln eines Alumosilikats mit einer sauren, wässrigen Magnesiumsalzlösung und
B1) Kalzinieren des mit wässriger Magnesiumsalzlösung behandelten Alumosilikats, aufweist. Unter Alumosilikaten sollen dabei Verbindungen verstanden werden, die sich im Wesentlichen formal aus Anteilen von Aluminiumoxid (Al₂O₃) und Siliziumdioxid (SiO₂) zusammensetzen. Die Alumosilikate können aber auch noch geringe Anteile an Alkali- oder Erdalkalimetalloxiden enthalten. In dem Verfahren können als Alumosilikate auch Zeolithe wie z. B. Zeolith A, X, Y, USY oder ZSM-5 oder amorphe Zeolithe (wie beispielsweise MCM 41 von Mobil Oil) eingesetzt werden. Die im Verfahren eingesetzten Alumosilikate können amorph oder kristallin sein. Geeignete kommerzielle Alumosilikate die als Ausgangsstoffe eingesetzt werden können sind beispielsweise Alumosilikate, die durch Fällung, Gelierung oder Pyrolyse hergestellt wurden. In dem Verfahren werden vorzugsweise Alumosilikate eingesetzt, die von 5 bis 40 Massen-%, bevorzugt 10 bis 35 Massen-% Aluminiumoxid und von 60 bis 95 Massen-%, bevorzugt von 65 bis 90 Massen Siliziumdioxid aufweisen (bezogen auf die Trockenmasse; Behandlung: Glühen bei 850 °C für 1 h). Die Bestimmung der Zusammensetzung der eingesetzten Alumosilikate bzw. der erhaltenen Katalysatoren kann z. B. durch klassische Analyse, Schmelzaufschluss mit Borax und RFA (Röntgenfluoreszenzanalyse), energiedispersive Röntgenanalyse, Flammen-spektroskopie (Al und Mg, nicht Si), nassem Aufschluss und anschließender ICP-OES (Optische Emissionsspektrometrie mit induktiv gekoppelten Hochfrequenzplasma) oder Atomabsorptionsspektroskopie erfolgen. Ein besonders bevorzugtes Alumosilikat, welches in dem Verfahren eingesetzt werden kann, weist einen formalen Anteil an Al₂O₃ von 13 Massen-% und einen Anteil an Siliziumdioxid von 76 Massen-% auf. Ein solches Alumosilikat wird von der Firma Grace Davison, unter der Bezeichnung Davicat O 701 angeboten.

Das Alumosilikat kann in den unterschiedlichsten Formen in dem Verfahren eingesetzt werden. So kann das Alumosilikat in Form von Formkörpern, wie z. B. Tabletten, Pellets, Granulat, Strängen oder Extrudaten eingesetzt werden. Das Alumosilikat kann aber auch als Alumosilikatpulver eingesetzt werden. Als Ausgangsmaterial kann von Pulvern mit unterschiedlicher mittlerer Korngröße und unterschiedlicher Korngrößenverteilung ausgegangen werden. Bevorzugt wird für die Herstellung von Formkörpern ein Alumosilikatpulver eingesetzt, bei dem 95 % der Partikel eine mittlere Korngröße von 5 bis 100 µm, vorzugsweise 10 bis 30 µm und besonders bevorzugt 20 bis 30 µm aufweisen. Die Bestimmung der Korngröße kann z. B. durch Laserbeugung mit einem Partikelanalysator der Firma Malvern, wie z. B. dem Mastersizer 2000 durchgeführt werden.

Zur Herstellung der wässrigen Magnesiumsalzlösung werden Magnesiumverbindungen verwendet werden, die wasserlöslich sind oder durch Zugabe einer Säure in wasserlösliche Verbindungen übergehen. Vorzugsweise werden als Salze die Nitrate eingesetzt. Bevorzugt werden Magnesiumsalzlösungen eingesetzt, die als Magnesiumsalze die Salze starker Mineralsäuren, wie beispielsweise Magnesiumnitrat-Hexahydrat oder Magnesiumsulfat-Heptahydrat aufweisen. Die eingesetzte saure wässrige Alkali- und/oder Erdalkalimetallsalzlösung weist vorzugsweise einen pH-Wert von kleiner 6, bevorzugt von kleiner 6 bis 3, und besonders bevorzugt von 5,5 bis 3,5 auf. Die Bestimmung des pH-Werts kann z. B. mit Hilfe einer Glaselektrode oder Indikatorpapier durchgeführt werden. Weist die Salzlösung einen pH-Wert auf, der größer oder gleich 6 ist, so kann der pH-Wert durch Zugabe einer Säure, vorzugsweise der Säure dessen Alkali- und/oder Erdalkalimetallsalz in der Lösung vorhanden ist, eingestellt werden. Vorzugsweise wird, wenn die Alkali- und/oder Erdalkalimetallsalzlösung als Salze die Nitrate aufweist, als Säure Salpetersäure eingesetzt. Der Magnesiumgehalt der eingesetzten Magnesiumsalzlösung beträgt vorzugsweise von 0,1 bis 3 Mol/l, bevorzugt von 0,5 bis 2,5 Mol/l.

Das Behandeln in Schritt A1) kann auf verschiedene Weisen erfolgen, die geeignet sind, das Alumosilikat mit der Magnesiumsalzlösung in Kontakt zu bringen. Mögliche Behandlungsmethoden sind z. B. Imprägnieren, Tränken, Besprühen oder Begießen des Alumosilikats mit der Magnesiumsalzlösung. Es kann vorteilhaft sein, wenn das Behandeln des Alumosilikats so erfolgt, dass die Magnesiumsalzlösung zumindest 0,1 bis 5 h bevorzugt von 0,5 bis 2 h auf das Alumosilikat einwirken kann. Eine solche Einwirkzeit kann insbesondere dann vorteilhaft sein, wenn das Behandeln durch einfaches Tränken erfolgt.

In einer bevorzugten Ausführungsform des Schrittes A1) des Verfahrens kann die Behandlung von Alumosilikat, insbesondere Alumosilikatformkörpern mit der Magnesiumsalzlösung beispielsweise durch Vakuumimprägnierung in einer dafür geeigneten Vakuumimprägnieranlage erfolgen. Bei dieser Art der Behandlung wird das Alumosilikat in der Vakuumimprägnieranlage zunächst evakuiert. Anschließend wird die Magnesiumsalzlösung bis über den oberen Rand der Trägerschüttung gesaugt, so dass das gesamte Alumosilikat mit der Lösung bedeckt ist. Nach einer Einwirkzeit, die vorzugsweise von 0,1 bis 10h, bevorzugt von 0,5 bis 2 h beträgt, wird die Lösung, die nicht vom Träger aufgenommen wurde, abgelassen.

In einer weiteren bevorzugten Ausführungsform des Schrittes A1) des Verfahrens kann die Behandlung von Alumosilikat, insbesondere Alumosilikatformkörpern mit der Alkali- und/oder Erdalkalimetallsalzlösung beispielsweise Besprühen oder Begießen des Alumosilikats erfolgen. Vorzugsweise erfolgt das Besprühen oder Begießen des Alumosilikats mit der Magnesiumsalzlösung in dem die Lösung auf das in einer Trommel rotierende Alumosilikat gesprüht oder gegossen wird. Die Behandlung kann in einem Zug erfolgen, d. h. zum Alumosilikat wird zu Beginn die gesamte Menge an Magnesiumsalzlösung in einem Schritt zugegeben. Die Salzlösung kann aber auch durch Besprühen oder Begießen in kleinen Portionen zudosiert werden, wobei der Zeitraum der Zugabe vorzugsweise von 0,1 bis 10 h und bevorzugt von 1 bis 3 h beträgt. Die Menge an Salzlösung wird vorzugsweise so bemessen, dass die gesamte Lösung vom Alumosilikat aufgenommen wird. Insbesondere das Tränken aber auch das Besprühen bzw. Begießen kann in üblichen technischen Apparaturen, wie beispielsweise Konusmischern oder Intensivmischern, wie sie z. B. von der Firma Eirich angeboten werden, durchgeführt werden.

Die Behandlung des Alumosilikats mit der Magnesiumsalzlösung in Schritt A1) kann in einem Schritt oder in mehreren Teilschritten erfolgen. Insbesondere ist es möglich, die Behandlung in zwei oder mehreren Teilschritten durchzuführen. In jedem der einzelnen Teilschritte kann jeweils die gleiche Magnesiumsalzlösung eingesetzt werden oder aber in jedem Teilschritt eine in der Konzentration verschiedene Magnesiumsalzlösung. Beispielsweise kann zum Alumosilikat zunächst nur ein Teil der Magnesiumsalzlösung zugesetzt werden und, gegebenenfalls nach einer Zwischentrocknung, bei gleicher oder anderer Temperatur die Restmenge der eingesetzten Magnesiumsalzlösung. Es ist nicht nur möglich, dass der Schritt A1) in zwei oder mehreren Teilschritten durchgeführt wird. Ebenfalls ist es möglich, dass das Verfahren mehrere Schritte A1) aufweist. Auch in diesem Fall können in den verschiedenen Schritten A1) gleiche oder unterschiedliche Magnesiumsalzlösung in Bezug auf die Konzentration eingesetzt werden.

Das Behandeln in Schritt A1) kann vorzugsweise bei einer Temperatur von 10 bis 120 °C, bevorzugt von 10 bis 90 °C, besonders bevorzugt von 15 bis 60 °C und ganz besonders bevorzugt bei einer Temperatur von 20 bis 40 °C durchgeführt werden.

Es kann vorteilhaft sein, wenn in Schritt A1) ein oder mehrere Additive dem Alumosilikat bzw. der Magnesiumsalzlösung zugegeben bzw. zugemischt werden. Solche Additive können z. B. Bindemittel, Gleitmittel oder Formgebungshilfsmittel sein. Ein geeignetes Bindemittel kann z. B. Boehmit oder Pesudo-Boehmit sein, wie es z. B. unter der Bezeichnung Disperal (einem Boehmit mit einem Al₂O₃-Gehalt von ca. 77 Massen-% (Rest Wasser und Spuren von Verunreinigungen)) von der Sasol Deutschland GmbH angeboten wird. Wird Boehmit, insbesondere Disperal als Bindemittel zugegeben, so wird dieses vorzugsweise als Gel zugegeben, welches z. B. durch Einrühren von 197 Massenteilen Disperal in 803 Massenteilen einer 1,28 Massen-%igen, wässrigen Salpetersäure, Gründliches Rühren bei 60 °C für 3 h, Abkühlen auf Raumtemperatur und Ergänzen von evtl. verdunstetem Wassererhalten erhalten werden kann. Als Formgebungshilfsmittel können beispielsweise Kieselsäuren, insbesondere pyrogene Kieselsäuren, wie sie z. B. von der Degussa AG unter der Bezeichnung Aerosil vertrieben werden, Bentonite, Tone, Kaolin, Kaolinit, ball clay und andere, dem Fachmann hierfür geläufige Stoffe eingesetzt werden. Als Gleitmittel, dessen Einsatz für die verbesserte Tablettierung vorteilhaft sein kann, kann beispielsweise Graphit zugesetzt werden.

Die Zugabe eines oder mehrerer der oben genannten Additive in Schritt A1) kann auf verschiedene Weisen erfolgen. Insbesondere kann die Zugabe während der Behandlung des Alumosilikats mit der Magnesiumsalzlösung erfolgen. Beispielweise können Alumosilikat, Additiv und Magnesiumsalzlösung in eine technische Apparatur gefüllt und anschließend innig vermischt werden. Eine andere Möglichkeit besteht darin, zuerst das Alumosilikat mit dem Additiv zu vermischen und anschließend die Magnesiumsalzlösung zuzusetzen. In einer weiteren Variante kann zum Alumosilikat Additiv und Magnesiumsalzlösung gleichzeitig zudosiert werden. Die Zugabe kann jeweils in einem Guss, in Portionen oder durch Sprühen erfolgen. Die Zugabezeit beträgt vorzugsweise weniger als 5 h bevorzugt weniger als 3 h. Es kann vorteilhaft sein, die Mischung für 0,1 bis 10 h, bevorzugt für 0,5 bis 3 h nachzumischen.

Das Herstellverfahren für den bevorzugt eingesetzten Katalysator weist zumindest einen Verfahrensschritt B1) auf, bei dem das mit Alkali- und/oder Erdalkalimetallsalzlösung behandelte Alumosilikat kalziniert wird. Die Kalzinierung erfolgt vorzugsweise in einem Gasstrom, beispielsweise in einem Gasstrom, der z. B. Luft, Stickstoff, Kohlendioxid und/oder eines oder mehrere Edelgase enthält oder aus einer oder mehrerer dieser Komponenten besteht. Bevorzugt erfolgt die Kalzinierung unter Verwendung von Luft als Gasstrom.

Die Kalzinierung in Verfahrensschritt B1) wird vorzugsweise bei einer Temperatur von 200 bis 1000 °C, bevorzugt von 300 bis 800 °C durchgeführt. Die Kalzinierung erfolgt vorzugsweise für eine Dauer von 0,1 bis 10 Stunden, bevorzugt von 1 bis 5 Stunden. Besonders bevorzugt wird die Kalzinierung bei einer Temperatur von 200 bis 1000 °C, vorzugsweise 300 bis 800 °C für 0,1 bis 10 Stunden, bevorzugt von 1 bis 5 Stunden durchgeführt.

Bevorzugt kann die technische Kalzinierung in einem Schachtofen durchgeführt werden. Die Kalzinierung kann jedoch auch in anderen bekannten technischen Apparaturen durchgeführt werden, wie beispielsweise Wirbelschichtkalzinierern, Drehrohröfen oder Hordenöfen.

Es kann vorteilhaft sein, wenn zwischen den Schritten A1) und B1) ein Schritt C1) durchgeführt wird, in dem das mit Magnesiumsalzlösung behandelte Alumosilikat getrocknet wird. Die Trocknung in Schritt C1) kann bei einer Temperatur von 100 bis 140 °C erfolgt. Vorzugsweise erfolgt die Trocknung in einem Gasstrom. Die Trocknung kann beispielsweise in einem Gasstrom, der z. B. Luft, Stickstoff, Kohlendioxid und/oder eines oder mehrere Edelgase enthält oder aus einer oder mehrerer dieser Komponenten besteht, durchgeführt werden. Durch den Zwischenschritt des Trocknens nach der Behandlung mit Alkali- und/oder Erdalkalimetallsalzlösung und vor dem Kalzinieren kann erreicht werden, dass bei der Kalzinierung keine großen Wasserdampfmengen freigesetzt werden. Zudem kann durch das Trocknen verhindert werden, dass durch beim Kalzinieren spontan verdampfendes Wasser die Form des Katalysators zerstört.

Je nachdem in welcher gewünschten Form der Katalysator vorliegen soll, kann es vorteilhaft sein, das Herstellungsverfahren durch zusätzliche Verfahrensschritte entsprechend anzupassen. Soll mit dem Verfahren beispielsweise pulverförmiger Katalysator hergestellt werden, so kann das Alumosilikat in Form von Alumosilikatpulver eingesetzt und z. B. in einem Konusmischer mit der Magnesiumsalzlösung behandelt (z. B. durch Imprägnieren) optional getrocknet und anschließend kalziniert werden. Ein pulverförmiger Katalysator kann aber auch dadurch hergestellt werden, dass ein Katalysatorformkörper durch Mahlen und Sieben zu pulverförmigem Katalysator verarbeitet wird.

Die Katalysatorformkörper können beispielsweise in Form von Strängen, Kugeln, Pellets oder Tabletten vorliegen. Um zum formgegebenen Katalysator (Katalysatorformkörper) zu gelangen, können abhängig von der jeweiligen Formgebungsvariante neben den Verfahrensschritten Behandlung, Trocknung, Kalzination, weitere Verfahrensschritte, wie z. B. Formgebung, Mahlung oder Siebung, durchgeführt werden. Formgebungshilfsmittel können an verschiedenen Stellen im Prozess eingebracht werden. Die Herstellung der Katalysatorformkörper kann auf unterschiedliche Weise erfolgen:

In einer ersten Ausführungsvariante können Katalysatorformformkörper dadurch erhalten werden, dass Alumosilikatformkörper mit einer sauren wässrigen Magnesiumsalzlösung behandelt, optional getrocknet und anschließend kalziniert werden.

In einer zweiten Ausführungsform kann ein Katalysatorkörper dadurch erhalten werden, dass ein Alumosilikatpulver, zunächst mit einer sauren wässrigen Magnesiumsalzlösung behandelt wird, dann gegebenenfalls getrocknet und anschließend kalziniert wird, und anschließend das erhaltene Katalysatorpulver durch in der Technik übliche Verfahren wie beispielsweise Kompaktierung, Extrusion, Pelletierung, Tablettierung, Granulation oder Coating zu Katalysatorformkörpern verarbeitet wird. Für die Formgebung benötigte Additive, wie z. B. Bindemittel oder weitere Hilfsstoffe können dabei an verschiedenen Stellen im Herstellungsprozess, so z. B. im Verfahrensschritt A1) zugegeben werden. Beim Herstellen eines Formkörpers aus einem Alumosilikatpulver als Ausgangsmaterial kann von Pulvern mit unterschiedlicher mittlerer Korngröße und unterschiedlicher Korngrößenverteilung ausgegangen werden. Bevorzugt wird für die Herstellung von Formkörpern ein Alumosilikatpulver eingesetzt, bei dem 95 % der Partikel eine Korngröße von 5 bis 100 µm, vorzugsweise von 10 bis 30 µm und besonders bevorzugt von 20 bis 30 µm aufweisen (Bestimmt durch Laserbeugung, siehe oben).

In einer dritten Ausführungsform des Verfahrens können Pellets des Katalysators dadurch erhalten werden, dass in Verfahrensschritt A1) ein Alumosilikatpulver mit einer sauren wässrigen Magnesiumsalzlösung behandelt, optional getrocknet (Verfahrensschritt C1)) und anschließend in Verfahrenschritt B1) kalziniert wird und das so erhaltene Katalysatorpulver, z. B. in einem Eirichmischer, unter Zugabe von Bindemittel pelletiert wird und die erhaltenen Pellets in einem weiteren Verfahrensschritt C1) getrocknet und anschließend in einem weiteren Verfahrensschritt B1) kalziniert werden.

In einer vierten Ausführungsform des Herstellverfahrens können Pellets des Katalysators dadurch erhalten werden, dass in Verfahrensschritt A1) ein Alumosilikatpulver, Bindemittel und saure wässrige Magnesiumsalzlösung gemischt werden und das so behandelte Alumosilikatpulver, z. B. in einem Eirichmischer, pelletiert wird und die erhaltenen feuchten Pellets in Verfahrensschritt C1) getrocknet und anschließend in Verfahrensschritt B1) im Gasstrom kalziniert werden.

In einer fünften Ausführungsform des Herstellverfahrens können Tabletten des Katalysators dadurch erhalten werden, dass in Verfahrensschritt A1) ein Alumosilikatpulver, Bindemittel, optional Gleitmittel und saure wässrige Magnesiumsalzlösung gemischt werden und das so behandelte Alumosilikatpulver, z. B. in einem Eirichmischer, zu Mikropellets vorzugsweise mit einem mittleren Durchmesser 0,5 bis 10 mm, bevorzugt 1 bis 5 mm und besonders bevorzugt von 1 bis 3 mm (Bestimmung der Korngröße kann z. B. durch Siebanalyse erfolgen) pelletiert wird und die erhaltenen feuchten Pellets in Verfahrensschritt C1) getrocknet und anschließend optional in Verfahrensschritt B1) im Gasstrom kalziniert werden. Die erhaltenen Pellets können dann, falls in Verfahrensschritt A1) noch nicht geschehen mit einem Gleitmittel, wie z. B. Graphit, vermischt und anschließend auf einer handelsüblichen Tablettenpresse, z. B. einer Rundläuferpresse, tablettiert werden. Die Tabletten können dann, falls noch kein Verfahrensschritt B1) durchgeführt wurde, in Verfahrensschritt B1) kalziniert werden.

In einer sechsten Ausführungsform des Herstellverfahrens können Tabletten des Katalysators dadurch erhalten werden, dass vorgeformte Formkörperkatalysatoren, wie sie z. B. als Pellets in Ausführungsform drei oder vier erhaltenen werden können, gemahlen und das erhaltene Granulat/Pulver gesiebt wird, so dass ein tablettierfähiges Granulat von Katalysator erhalten wird, und diesem Granulat Gleitmittel zugemischt wird. Das so vorbereitete Granulat kann anschließend tablettiert werden. Die Tabletten können dann, falls noch kein Verfahrensschritt B1) durchgeführt wurde, in Verfahrensschritt B1) kalziniert werden. Das Zumischen eines Gleitmittels kann entfallen, wenn bereits bei der Herstellung der Pellets, z. B. in Verfahrenschritt A1) ein Gleitmittel zugefügt wurde.

In einer siebten Ausführungsform des Verfahrens können mit dem Katalysator beschichtete Materialien/Träger hergestellt werden. Bei dieser Ausführungsform wird zunächst ein Katalysatorpulver dadurch hergestellt werden, dass in Verfahrensschritt A1) ein Alumosilikatpulver mit einer sauren wässrigen Magnesiumsalzlösung behandelt, optional getrocknet (Verfahrensschritt C1)) und optional kalziniert (Verfahrenschritt B1)) wird. Das so erhaltene Katalysatorpulver, wird anschließend in einem Supensionsmittel, wie z. B. Wasser oder Alkohol suspendiert, wobei gegebenenfalls ein Bindemittel der Suspension zugegeben werden kann. Die so hergestellte Suspension kann dann auf jedes beliebige Material aufgebracht werden. Nach dem Aufbringen wird optional getrocknet (Verfahrensschritt C1)) und anschließend kalziniert (Verfahrenschritt B1)). Auf diese Weise lassen sich mit dem bevorzugten Katalysator beschichtete Materialien/Träger bereitstellen. Solche Materialien/Träger können z. B. Metallplatten oder -gewebe, wie sie als Einbauten in Reaktoren oder Kolonnen, insbesondere Reaktivdestillationskolonnen verwendet werden können, oder auch Metall-, Glas- oder Keramikkugeln oder Kugeln von anorganischen Oxiden sein.

In einer achten Ausführungsform des Herstellverfahrens können Extrudate des Katalysators, dadurch erhalten werden, dass in Verfahrensschritt A1) ein Alumosilikatpulver, saure wässrige Alkali- und/oder Erdalkalimetallsalzlösung, Bindemittel beispielsweise Disperal und weitere für die Extrusion übliche Formhilfsmittel beispielsweise Tone wie Bentonit oder Attapulgit in einem Kneter oder Eirich-Mischer gemischt werden und in einem Extruder zu Extrudaten vorzugsweise mit einem mittleren Durchmesser von 0,5 bis 10 mm, bevorzugt von 1 bis 5 mm und besonders bevorzugt von 1 bis 3 mm extrudiert werden und die erhaltenen feuchten Extrudate optional in Verfahrensschritt C1) getrocknet und anschließend in Verfahrensschritt B1) im Gasstrom kalziniert werden.

Der Reaktionsdruck beträgt in Schritt b) vorzugsweise von 0,1 bis 10 MPa(abs), bevorzugt zwischen 0,5 und 0,8 MPa(abs). Die Spaltung wird vorzugsweise bei einer WHSV (weight hourly space velocity) von 0,1 bis 5 h⁻¹, bevorzugt von 1 bis 3 h⁻¹ (kg MTBE je kg Katalysator je Stunde) durchgeführt. Der MTBE-Umsatz im geraden Durchgang beträgt vorzugsweise 90 bis 95 %. Die Spaltung kann in üblichen Reaktoren, wie z. B. in einem Rohrreaktor, Rohrbündelreaktor, Schachtofen oder Wirbelbettreaktor oder einer Kombination davon durchgeführt werden.

Vorzugsweise wird die Spaltung in der Gasphase in einem Reaktor, der mit einem Heizmantel ausgestattet ist und der mit einem flüssigen Wärmeträger beheizt wird, durchgeführt, wobei die Spaltung so durchgeführt wird, dass der Temperaturabfall in der Katalysatorzone/Reaktionszone an jeder beliebigen Stelle in Bezug auf die Eingangstemperatur von kleiner 50 °C, vorzugsweise kleiner 40 °C und besonders bevorzugt von 1 bis 30 °C beträgt, dass das Reaktionsgemisch im Reaktor und der Wärmeträger im Mantel im Gleichstrom durch den Reaktor strömen und dass die Temperaturdifferenz des Wärmeträgers zwischen Zulaufstelle zum Reaktor und Ablauf aus dem Reaktor weniger als 40 °C beträgt. Der maximale Temperaturabfall kann durch zahlreiche Parameter, wie z. B. durch die Temperatur des zum Heizen verwendeten Wärmeträgers sowie durch die Geschwindigkeit, mit der der Wärmträger durch den Mantel strömt, eingestellt werden.

Vorzugsweise liegt die Eingangstemperatur des gasförmigen Edukts, insbesondere bei dieser bevorzugten Ausführungsform des erfindungsgemäßen Verfahrenschrittes b), über 200 °C, bevorzugt über 230 °C und besonders bevorzugt über 250 °C. Die Eingangstemperatur des Edukts kann in einem dem Reaktor vorgeschalteten Aufheizer eingestellt werden. Bei Verwendung von frischem Katalysator, insbesondere bei der Verwendung von frischem Magnesiumoxid/Aluminiumoxid/Siliziumoxid-Katalysator bei der MTBE-Spaltung liegt die Eingangstemperatur bevorzugt zwischen 250 bis 270 °C. Im Laufe des Betriebes kann es vorteilhaft sein, mit zunehmender Desaktivierung des Katalysators zur Konstanthaltung des Umsatzes die Eingangstemperatur bis auf 400 °C anzuheben. Kann der Umsatz bei Erreichen von 400 °C nicht mehr gehalten werden, so kann es vorteilhaft sein, den Katalysator ganz oder teilweise zu ersetzen.

Der Reaktor wird, insbesondere bei dieser bevorzugten Ausführungsform des erfindungsgemäßen Verfahrenschrittes b), vorzugsweise mit einer Raumgeschwindigkeit (Weight Hourly Space Velocity (WHSV) in Kilogramm Edukt je Kilogramm Katalysator je Stunde) von 0,1 bis 5 h⁻¹, insbesondere von 1 bis 3 h⁻¹ im geraden Durchgang betrieben.

Der Reaktor kann, insbesondere bei dieser bevorzugten Ausführungsform des erfindungsgemäßen Verfahrenschrittes b), in jeder beliebigen Raumrichtung angeordnet sein. Weist der Reaktor Reaktorrohre auf, so können diese ebenfalls in jede beliebige Raumrichtung weisen. Bevorzugt ist der Reaktor jedoch derart aufgestellt, dass der Reaktor bzw. die Reaktorrohre vertikal ausgerichtet sind. Bei einem senkrecht ausgerichteten Reaktor wird der Wärmeträger bevorzugt an der höchsten Stelle oder in der Nähe der höchsten Stelle des Mantels zugeführt und an der tiefsten Stelle oder in der Nähe der tiefsten Stelle des Reaktors abgezogen oder umgekehrt. Das Reaktionsgemisch in der Reaktionszone und der Wärmeträger im Mantel durchströmen den Reaktor vorzugsweise in gleicher Richtung. Besonders bevorzugt durchströmen der Wärmeträger und das Reaktionsgemisch den Mantel des Reaktors bzw. die Reaktionszone des Reaktors von oben nach unten.

Um eine gleichmäßigere Beheizung der Reaktionszone zu erreichen, kann es vorteilhaft sein, den Wärmeträger nicht nur an einer Stelle, sondern an mehreren Stellen in etwa gleicher Höhe in den Reaktor einzuspeisen. Um bei der Verwendung eines Rohrbündelreaktors einen größeren Temperaturabfall in den mittleren Rohren im Vergleich zu Randrohren zu vermeiden, kann es vorteilhaft sein, in dem Zulauf oder in den Zuläufen für den Wärmeträger Düsen vorzusehen, die den Transport des Wärmeträgers zu den mittleren Rohren begünstigen. Auf diese Weise können Temperaturschwankungen über den Querschnitt des Rohrbündels vermieden werden.

Der Wärmeträger kann an einer oder mehreren Stelle(n) den Reaktor verlassen. Wird der Reaktor vom Wärmeträger von oben nach unten durchströmt, ist durch konstruktive Maßnahmen sicherzustellen, dass die Reaktionszonen, wie z. B. die Reaktionsrohre, vollständig mit Wärmeträger umspült werden.

Der Wärmeträger kann außerhalb des Reaktors durch direkte oder indirekte Beheizung auf die gewünschte Temperatur gebracht und durch den Reaktor gepumpt werden.

Als Wärmeträger können Salzschmelzen, Wasser oder Wärmeträgeröle verwendet werden. Für den Temperaturbereich von 200 bis 400 °C ist die Verwendung von Wärmeträgerölen vorteilhaft, da Heizkreisläufe mit ihnen im Vergleich zu anderen technischen Lösungen einen geringeren Kapitaleinsatz erfordern. Wärmeträgeröle, die eingesetzt werden können, sind beispielsweise solche, die unter den Handelsnamen Marlotherm (z. B. Marlotherm SH der Sasol Olefins & Surfactants GmbH), Diphyl (Fa. Bayer), Dowtherm (Fa. Dow) oder Therminol (Fa. Therminol) vertrieben werden. Diese synthetisch hergestellten Wärmeträgeröle basieren im Wesentlichen auf thermisch stabilen Ringkohlenwasserstoffen.

Vorzugsweise wird der Wärmeträger mit einer Temperatur, die 10 bis 40 °C, bevorzugt 10 bis 30 °C höher ist, als die Temperatur des in den Reaktor strömenden Edukts, in den Heizmantel des Reaktors geleitet. Die Temperaturdifferenz des flüssigen Wärmeträgers über den Reaktor, also zwischen Eintrittstemperatur des Wärmeträgers bei Eintritt in den Heizmantel und der Austrittstemperatur des Wärmeträgers bei Austritt aus dem Heizmantel beträgt vorzugsweise weniger als 40 °C, bevorzugt weniger als 30 °C und besonders bevorzugt von 10 bis 25 °C. Die Temperaturdifferenz kann durch den Massenfluss des Wärmeträgers pro Zeiteinheit (Kilogramm je Stunde) durch den Heizmantel eingestellt werden.

Die bevorzugte Ausführungsform des erfindungsgemäßen Verfahrensschrittes b) kann in allen geeigneten Reaktoren, die mit einem Heizmantel ausgestattet sind und die mit einem flüssigen Wärmeträger beheizt werden können durchgeführt werden. Solche Reaktoren weisen eine den Katalysator aufweisende Reaktionszone (Katalysatorzone) auf, die räumlich von einem Heizmantel, durch den der Wärmeträger strömt, getrennt ist. Vorzugsweise wird das erfindungsgemäße Verfahren in einem Plattenreaktor, in einem Rohrreaktor, in mehreren parallel zueinander geschalteten Rohrreaktoren oder Plattenreaktoren oder in einem Rohrbündelreaktor durchgeführt. Bevorzugt wird das erfindungsgemäße Verfahren in einem Rohrbündelreaktor durchgeführt.

Es sei darauf hingewiesen, dass die Hohlkörper, in denen sich der Katalysator befindet, nicht nur Rohre im üblichen Sprachgebrauch sein müssen. Die Hohlkörper können auch nicht kreisförmige Querschnitte aufweisen. Sie können beispielsweise elliptisch oder dreieckig sein.

Die für den Bau des Reaktors verwendeten Materialien, insbesondere das Material, welches die Reaktionszone von dem Heizmantel trennt, weist vorzugsweise einen hohen Wärmeleitfähigkeitskoeffizienten (größer 40 W/(m · K)) auf. Bevorzugt wird als Material mit hohem Wärmeleitfähigkeitskoeffizient Eisen oder eine Eisenlegierung, wie z. B. Stahl eingesetzt.

Wird das erfindungsgemäße Verfahren in einem Rohrbündelreaktor durchgeführt, so weisen die einzelnen Rohre vorzugsweise eine Länge von 1 bis 15 m, bevorzugt von 3 bis 9 m und besonders bevorzugt 5 bis 9 m auf. Die einzelnen Rohre in einem im erfindungsgemäßen Verfahren eingesetzten Rohrbündelreaktor weisen vorzugsweise einen inneren Durchmesser von 10 bis 60 mm, bevorzugt von 20 bis 40 mm und besonders bevorzugt von 24 bis 35 mm auf. Es kann vorteilhaft sein, wenn die einzelnen Rohre des im erfindungsgemäßen Verfahren eingesetzten Rohrbündelreaktors eine Dicke der Rohrwand von 1 bis 4 mm, vorzugsweise von 1,5 bis 3 mm aufweisen.

In einem in der bevorzugten Ausführungsform des erfindungsgemäßen Verfahrenschrittes b) eingesetzten Rohrbündelreaktor sind die Rohre vorzugsweise parallel angeordnet. Bevorzugt sind die Rohre gleichmäßig angeordnet. Die Anordnung der Rohre kann z. B. quadratisch, dreieckig oder rautenförmig sein. Besonders bevorzugt wird eine Anordnung, bei der die virtuell verbundenen Mittelpunkte von drei gegenseitig benachbarten Rohren ein gleichseitiges Dreieck bilden, d. h. die Rohre haben den gleichen Abstand. Bevorzugt wird das erfindungsgemäße Verfahren in einem Rohrbündelreaktor, in dem die Rohre einen Abstand von 3 bis 15 mm, besonders bevorzugt von 4 bis 7 mm zueinander aufweisen, durchgeführt.

Vorzugsweise wird die Spaltung in Schritt b) unter solchen Bedingungen durchgeführt, bei denen der Umsatz an MTBE größer als der Umsatz von 2-Methoxybutan ist. Auf diese einfache Weise kann nach Maßgabe der Erfindung sichergestellt werden, dass das Spaltprodukt eine Konzentration von weniger als 1000 Massen-ppm an linearen Butenen bezogen auf die C₄-Olefinfraktion aufweist, auch wenn der Kopfstrom II einen Anteil an 2-Methoxybutan (MSBE) von größer 1000 Massen-ppm in Bezug auf MTBE enthält. Je nach Anteil des im Kopfstrom II enthaltenen MSBE kann es notwendig sein, solche Bedingungen einzustellen, bei denen der Umsatz an MSBE deutlich, also z. B. um zumindest 50 % geringer ist als der Umsatz an MTBE. Diese Bedingungen können durch einfache Vorversuche ermittelt werden.

Die Hauptreaktion im Verfahrensschritt b) des erfindungsgemäßen Verfahrens ist die Spaltung von MTBE zu Isobuten und Methanol. Für den Fall, dass dem Einsatz-MTBE kein Rückführstrom aus der Spaltung zugemischt wird, weist das Spaltprodukt IV, je nach eingestelltem MTBE-Umsatz, vorzugsweise einen Rest-MTBE-Gehalt von 2 bis 29 Massen-% auf. Der Methanolgehalt beträgt vorzugsweise von 25 bis 35 Massen-%, der Isobutengehalt vorzugsweise von 44 bis 61 Massen-%.

Für den Fall, das nicht umgesetztes MTBE in einer sich an die Spaltung anschließenden Destillation vom Spaltprodukt abgetrennt und in die Spaltung zurückgeführt wird, ändern sich entsprechend dem Methanolgehalt des rückgeführten MTBEs die Zusammensetzungen. Als Nebenreaktionen können bei der Spaltung die Bildung von Diisobuten aus Isobuten und die Reaktion von Methanol zu Dimethylether auftreten. Im Edukt enthaltenes 2-Methoxybutan kann außerdem teilweise zu linearen Butenen und enthaltener Tertiärbutylalkohol (TBA) kann zu Isobuten und Wasser gespalten werden. Daher können als weitere, durch Reaktion im Verfahrensschritt b) entstandene Komponenten u. a. Diisobuten, Dimethylether, lineare Butene und Wasser im Spaltprodukt IV enthalten sein.

### Verfahrensschritt c)

Um das Spaltproduktgemisch weiter aufzuarbeiten kann es vorteilhaft sein, wenn das Spaltprodukt IV in einem weiteren Destillationsschritt c) in einen Isobuten aufweisenden Kopfstrom V und einen nicht umgesetztes MTBE aufweisenden Sumpfstrom VI getrennt wird. Die destillative Auftrennung des Spaltprodukts IV in einen Isobuten aufweisenden Kopfstrom V und einen nicht umgesetztes MTBE aufweisenden Sumpfstrom VI gemäß Verfahrensschritt c) erfolgt in zumindest einer Kolonne, bevorzugt in genau einer Destillationskolonne.

Eine in Verfahrensschritt c) bevorzugt eingesetzte Destillationskolonne weist vorzugsweise von 20 bis 55 theoretische Trennstufen, bevorzugt von 25 bis 45 und besonders bevorzugt von 30 bis 40 theoretische Trennstufen auf. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 1. Der Betriebsdruck der Kolonne K2 kann vorzugsweise zwischen 0,1 und 2,0 MPa(abs) eingestellt werden. Um einen Kompressor zu sparen, kann es vorteilhaft sein, die Kolonne bei einem niedrigeren Druck als den Druck, mit dem der Spaltreaktor R in Verfahrensschritt b) betrieben wird, zu betreiben. Um Isobuten gegen Kühlwasser kondensieren zu können, ist ein Druck von ca. 0,5 MPa(abs) notwendig. Wird der Spaltung in Verfahrensschritt b) beispielsweise bei einem Druck von 0,65 MPa (abs) betrieben, kann es vorteilhaft sein, wenn die Destillationskolonne des Verfahrensschrittes c) mit einem Betriebsdruck von 0,55 bis 0,6 MPa (abs) durchgeführt wird. Zur Beheizung des Verdampfers kann z. B. 0,4 MPa-Dampf eingesetzt werden. Das Sumpfprodukt VI enthält vorzugsweise nicht umgesetztes MTBE, Methanol sowie gegebenenfalls Nebenprodukte, wie beispielsweise Diisobuten und 2-Methoxybutan. Das Kopfprodukt ist vorzugsweise Isobuten mit einer Reinheit größer 95 Massen-%, bezogen auf das gesamte Kopfprodukt.

Optional kann der Verfahrensschritt c) in zumindest einer als Reaktivdestillationskolonne ausgeführten Kolonne durchgeführt werden. Diese Ausführungsform des erfindungsgemäßen Verfahrens hat den Vorteil, dass der MTBE-Umsatz im gesamten Verfahren dadurch erhöht werden kann, dass ein Teil des in Verfahrensschritt b) nicht umgesetzten MTBEs im Reaktionsteil der Reaktivdestillationskolonne des Verfahrensschrittes c) zu Isobuten und Methanol gespalten wird.

Als Katalysatoren können im Reaktionsteil der Reaktivdestillationskolonne alle Katalysatoren eingesetzt werden, die zur Spaltung von MTBE geeignet sind. Bevorzugt werden als Katalysatoren saure Katalysatoren eingesetzt. Eine besonders bevorzugte Gruppe von sauren Katalysatoren für den Einsatz im Reaktionsteil der Reaktivdestillationskolonne sind feste, saure Ionenaustauscherharze, insbesondere solche mit Sulfonsäuregruppen. Geeignete saure Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Die Eigenschaften dieser Harze, insbesondere spezifische Oberfläche, Porosität, Stabilität, Quellung bzw. Schrumpfung und Austauschkapazität, können durch den Herstellprozess variiert werden.

Im Reaktionsteil der Reaktivdestillationskolonne können die Ionenaustauscherharze in ihrer H-Form eingesetzt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlyst 46, Amberlite IR-120, Amberlite 200, Dowex 50, Lewatit SPC 118, Lewatit SPC 108, K2611, K2621, OC 1501.

Das Porenvolumen der eingesetzten Ionenaustauscherharze beträgt vorzugsweise von 0,3 bis 0,9 ml/g, insbesondere von 0,5 bis 0,9 ml/g. Die Korngröße des Harzes beträgt vorzugsweise von 0,3 mm bis 1,5 mm, bevorzugt von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauschers beträgt, bezogen auf die Lieferform, vorzugsweise von 0,7 bis 2,0 eq/1, insbesondere von 1,1 bis 2,0 eq/1.

Im Reaktionsteil einer optional als Reaktivdestillationskolonne ausgeführten Kolonne im Verfahrensschritt c) kann der Katalysator entweder in der Packung integriert sein, wie beispielsweise in KataMax® (wie in EP 0 428 265 beschrieben) oder KataPak® (wie in EP 0 396 650 oder DE 298 07 007.3 U1 beschrieben) Packungen, oder auf Formkörpern aufpolymerisiert sein (wie in US 5,244,929 beschrieben).

Bevorzugt weist die Reaktivdestillationskolonne oberhalb der Katalysatorpackung einen Bereich rein destillativer Trennung auf. Bevorzugt weist die Zone oberhalb der Katalysatorpackung 5 bis 25, insbesondere 5 bis 15 theoretische Trennstufen auf. Die Trennzone unterhalb des Katalysators umfasst vorzugsweise von 5 bis 35, bevorzugt von 5 bis 25 theoretische Trennstufen. Der Zulauf zur Reaktivdestillationskolonne kann oberhalb oder unterhalb, vorzugsweise oberhalb der Katalysatorzone erfolgen.

Die Umsetzung des MTBEs zu Isobuten und Methanol erfolgt in der Reaktivdestillation vorzugsweise in einem Temperaturbereich von 60 bis 140 °C, bevorzugt bei 80 bis 130 °C, besonders bevorzugt bei 90 bis 110 °C (Temperatur im Bereich der Kolonne, in der sich der Katalysator befindet; die Sumpftemperatur kann deutlich höher liegen).

Für den Betriebsdruck der Reaktivdestillationskolonne können prinzipiell ähnliche Betriebsbedingungen wie für die oben beschriebene Ausführung als reine Destillationskolonne gewählt werden. So wird vorzugsweise ein Betriebsdruck der Reaktivdestillationskolonne von 0,1 und 1,2 MPa(abs) eingestellt. Um einen Kompressor zu sparen, kann es vorteilhaft sein, die Kolonne bei einem niedrigeren Druck als den Druck, mit dem der Spaltreaktor R in Verfahrensschritt b) betrieben wird, zu betreiben. Um Isobuten gegen Kühlwasser kondensieren zu können, ist ein Druck von ca. 0,5 MPa(abs) notwendig. Wird der Spaltung in Verfahrensschritt b) beispielsweise bei einem Druck von 0,65 MPa (abs) betrieben, kann es vorteilhaft sein, wenn die Destillationskolonne des Verfahrensschrittes c) mit einem Betriebsdruck von 0,55 bis 0,6 MPa (abs) durchgeführt wird. Zur Beheizung des Verdampfers kann z. B. Dampf eingesetzt werden.

Die hydraulische Belastung in der katalytischen Packung der Kolonne beträgt vorzugsweise von 10 % bis 110 %, bevorzugt von 20 % bis 70 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden. (Vauck/Müller, "Grundoperationen chemischer Verfahrenstechnik", S. 626, VEB Deutscher Verlag für Grundstoffindustrie.)

Auch bei einer Ausführung der Kolonne in Verfahrensschritt c) als Reaktivdestillationskolonne wird vorzugsweise ein Sumpfprodukt VI erhalten, das nicht umgesetztes MTBE und Methanol sowie gegebenenfalls Nebenprodukte, wie beispielsweise Diisobuten und 2-Methoxybutan enthält. Das Kopfprodukt weist vorzugsweise Isobuten mit einer Reinheit größer 95 Massen-% auf.

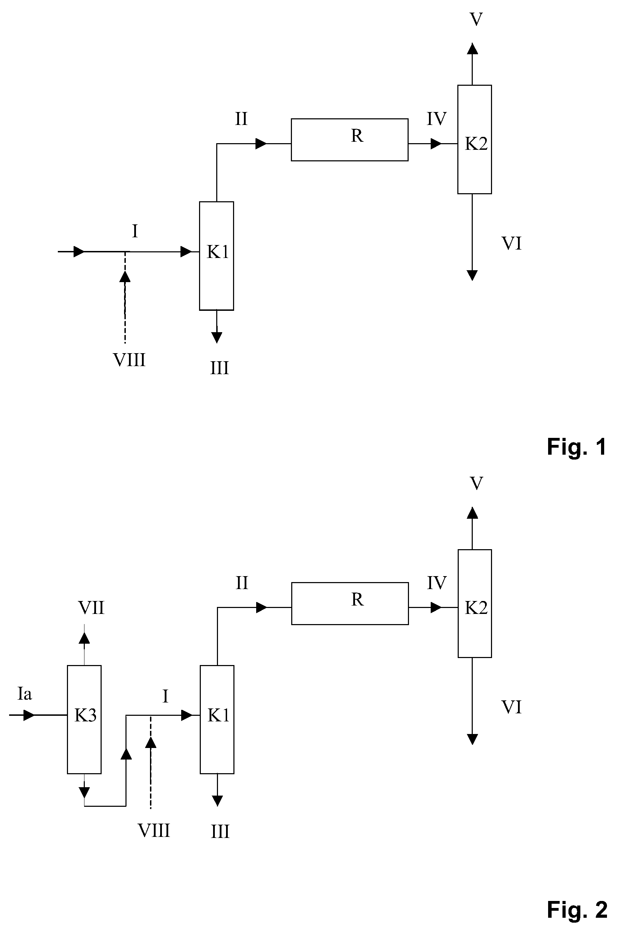

Das in Verfahrensschritt c) erhaltene Sumpfprodukt VI enthält das im Verfahrensschritt b) nicht umgesetzte MTBE, und den größten Teil des bei der Spaltung des MTBE entstandenen Methanols. Gegebenenfalls kann das Sumpfprodukt Nebenprodukte, wie beispielsweise Diisobuten und/oder 2-Methoxybutan enthalten. Für die Verwendung bzw. Aufarbeitung dieses Stroms VI gibt es verschiedene Möglichkeiten. Steht die MTBE-Spaltanlage im Verbund mit einer Anlage zur Herstellung von MTBE, so kann Strom VI in die MTBE-Anlage, vorzugsweise in den Syntheseteil, gefahren werden. Dies gilt selbst dann, wenn die MTBE-Anlage nur gerade so viel MTBE erzeugt, wie für die Spaltung benötigt wird und somit im Syntheseteil kein weiterer Auslass für schwersiedende Komponenten vorhanden ist. Eine zweite Möglichkeit besteht darin, aus dem Strom VI den größten Teil des Methanols destillativ abzutrennen und den Rest in den Verfahrensschritt a) zurückzufahren (Strom VIII in Fig. 2). Eine weitere Möglichkeit liegt darin, aus dem Strom VI Methanol, die Nebenprodukte und 2-Methoxybutan destillativ abzutrennen und das zurückbleibende MTBE in den Verfahrensschritt b) zurückzufahren. Die beiden letzteren Möglichkeiten sind insbesondere für allein stehende Anlagen (stand alone plants), bei denen angeliefertes MTBE eingesetzt wird, vorteilhaft.

Das Kopfprodukt V, das in Verfahrensschritt c) erhalten wird und das vorzugsweise zu größer 95 Massen-% aus Isobuten besteht, kann direkt als Verkaufsprodukt eingesetzt werden oder weiter aufgereinigt werden.

Da Isobuten mit Methanol ein Minimum-Azeotrop bildet, enthält das in Verfahrensschritt c) erhaltene Kopfprodukt V neben dem Hauptprodukt Isobuten insbesondere Methanol. Als weitere Komponenten können im Kopfprodukt V z. B. Dimethylether, welches z. B. durch Kondensation von Methanol entstanden sein kann, und lineare Butene (1-Buten, cis-2-Buten, trans-2-Buten), welches z. B. durch Zersetzung von 2-Methoxybutan entstanden sein können, und Wasser enthalten sein.

Aus dem Kopfprodukt V kann ein Teil des Dimethylethers optional bereits in Verfahrensschritt c) abgetrennt werden, indem der Kondensator an der Destillationskolonne bzw.

Reaktivdestillationskolonne als Partialkondensator betrieben wird. In diesem kann die im Kopfprodukt enthaltene C₄-Fraktion kondensiert werden und ein Teil des im Kopfprodukt V enthaltenen Dimethylethers gasförmig abgezogen werden.

Der Gehalt an linearen Butenen im in Verfahrensschritt c) erhaltenen Kopfprodukt V beträgt bezogen auf die C₄-Olefinfraktion vorzugsweise weniger als 1000 Massen-ppm. Dabei beträgt der Gehalt an 1-Buten in der Kopffraktion V bezogen auf die C₄-Olefinfraktion vorzugsweise weniger als 500 Massen-ppm. Der Gehalt an 2-Butenen (Summe der beiden 2-Butene) beträgt bezogen auf die C₄-Olefinfraktion vorzugsweise ebenfalls weniger als 500 Massen-ppm.

### Verfahrensschritt d) Isobutenaufarbeitung

Marktgängige Isobutenqualitäten sind üblicherweise praktisch frei von Methanol. Das Methanol kann aus dem in Verfahrensschritt c) erhaltenen Strom V nach an sich bekannten Verfahren, beispielsweise durch Extraktion, abgetrennt werden. Die Extraktion von Methanol aus Strom V kann beispielsweise mit Wasser oder einer wässrigen Lösung als Extraktionsmittel z. B. in einer Extraktionskolonne durchgeführt werden. Vorzugsweise wird die Extraktion mit Wasser oder einer wässrigen Lösung in einer Extraktionskolonne durchgeführt, die vorzugsweise von 4 bis 16 theoretische Trennstufen aufweist. Das Extraktionsmittel wird in Bezug auf den zu extrahierenden Strom vorzugsweise im Gegenstrom durch die Extraktionskolonne geführt. Die Extraktion wird vorzugsweise bei einer Temperatur von 15 bis 50 °C, bevorzugt 25 bis 40 °C durchgeführt. Beispielweise kann bei der Verwendung einer Extraktionskolonne mit mehr als 6 theoretischen Trennstufen, die bei einem Druck von 0,9 MPa_{(abs)} und einer Temperatur von 40°C betrieben wird, ein wassergesättigtes Isobuten mit einem Isobutengehalt von über 99 Massen-% erhalten werden.

Der bei der Extraktion erhaltene methanolhaltige Wasserextrakt kann destillativ in Wasser und Methanol getrennt werden. Das Wasser kann als Extraktionsmittel in die Extraktionsstufe zurückgeführt werden. Das Methanol kann für übliche technische Synthesen, beispielsweise Veresterungen oder Veretherungen genutzt werden.

Der feuchte Isobutenstrom aus der Extraktionskolonne kann in einer weiteren Destillationskolonne von Dimethylether und Wasser getrennt und zu trockenem Isobuten aufgearbeitet werden. Das trockene Isobuten wird dabei als Sumpfprodukt erhalten. Im Kondensationssystem am Kopf der Kolonne wird nach Phasentrennung Wasser flüssig und Dimethylether gasförmig abgezogen. Eine für die Trocknung bevorzugt eingesetzte Destillationskolonne weist vorzugsweise von 30 bis 80 theoretische Trennstufen, bevorzugt von 40 bis 65 theoretische Trennstufen auf. Das Rücklaufverhältnis beträgt, in Abhängigkeit von der realisierten Stufenzahl und der erforderlichen Reinheit des Isobutens, vorzugsweise kleiner 60, bevorzugt kleiner 40. Der Betriebsdruck der Kolonne K2 kann vorzugsweise zwischen 0,1 und 2,0 MPa(abs) eingestellt werden.

Eine Aufbereitung von Isobuten durch Extraktion und Destillation ist z. B. in DE 102 38 370 ausführlich beschrieben.

Das derart gewonnene Isobuten kann z. B. die in Tabelle 1 aufgeführte Zusammensetzung aufweisen:

**Tabelle 1: Typische Zusammensetzung von marktgängigem Isobuten**

| Massenanteile [kg/kg] | |
|---|---|
| C₃-Kohlenwasserstoffe | <0,000100 |
| Butane | <0,001000 |
| Isobuten | >0,999000 |
| 1-Buten | <0,000500 |
| 2-Butene | <0,000500 |
| Methanol | <0,000030 |
| C₅-Kohlenwasserstoffe | <0,000500 |
| Wasser | <0,000050 |

Je nach Reinheitsanforderungen sind aber bei Bedarf auch geringere Konzentrationen der Nebenkomponenten denkbar.

Das mit dem erfindungsgemäßen Verfahren hergestellte Isobuten kann z. B. zur Herstellung von Methallylchlorid, Methallylsulfonate, Methacrylsäure oder Methylmethacrylat eingesetzt werden. Insbesondere kann es vorteilhaft sein, wenn sowohl das Methanol als auch das Isobuten aus dem Spaltprodukt abgetrennt wird, sowohl das Methanol als auch das Isobuten zur Herstellung von Methylmethacrylat einzusetzen. Ein solches Verfahren zur Herstellung von Methylmethacrylat wird beispielsweise in EP 1 254 887 beschrieben, auf welches ausdrücklich verwiesen wird.

### Einsatzstoff

Als MTBE aufweisender Strom I kann in Verfahrensschritt a) MTBE unterschiedlicher Qualität eingesetzt werden. Insbesondere können technisches MTBE verschiedener Qualitäten oder Gemische aus technischem MTBE und Methanol als Strom I eingesetzt werden. Technisches MTBE (Kraftstoffqualität) ist der bevorzugte Einsatzstoff. Die Tabelle 2 zeigt beispielsweise die typische Zusammensetzung eines technischen MTBE der OXENO Olefinchemie GmbH.

**Tabelle 2: Typische Zusammensetzung von technischem MTBE (Kraftstoffqualität) der Oxeno.**

| Massenanteile [kg/kg] | |
|---|---|
| 1-Buten | 0,000080 |
| 2-Butene | 0,000920 |
| Pentane | 0,001500 |
| MTBE | 0,978000 |
| 2-Methoxybutan | 0,003000 |
| Methanol | 0,008500 |
| tert.-Butanol | 0,003000 |
| Wasser | 0,000050 |
| Diisobuten | 0,003300 |

Technisches MTBE kann nach bekannten Verfahren durch Umsetzung von C₄-Kohlenwasserstoffgemischen, aus denen die mehrfach ungesättigten Kohlenwasserstoffe weitgehend entfernt worden sind, beispielsweise Raffinat I oder selektiv hydriertes Crack-C₄, mit Methanol hergestellt werden. Ein Verfahren zur Herstellung von MTBE wird beispielsweise in DE 101 02 062 beschrieben.

Als Strom I kann im erfindungsgemäßen Verfahren auch ein Gemisch aus technischem MTBE und einem destillativ abgetrennten Teilstrom des Spaltproduktes IV eingesetzt werden. Dieser Teilstrom kann insbesondere der Strom sein, der in Verfahrenschritt c) als Sumpfprodukt VI erhalten wird oder ein Strom, der durch Aufbereitung des in Verfahrensschritt c) erhaltenen Sumpfstroms VI erhalten werden kann.

In dem erfindungsgemäßen Verfahren kann es besonders vorteilhaft sein, wenn als Strom I ein MTBE aufweisender Strom eingesetzt wird, der ganz oder teilweise durch Abtrennen von Leichtsiedern in einem optionalen Verfahrensschritt e) aus einem MTBE aufweisenden Strom Ia erhalten wird. Auch diesem Strom I kann der destillativ abgetrennte Teilstrom des Spaltproduktes IV zugegeben werden.

Das Abtrennen von Leichtsiedern kann insbesondere dann vorteilhaft sein, wenn der MTBE aufweisende Strom Ia z. B. C₄- oder C₅-Kohlenwasserstoffe aufweist. Das Abtrennen der Leichtsieder aus Strom Ia in dem optionalen Verfahrensschritt e) kann vorzugsweise in einer Destillationskolonne erfolgen. Die Destillationskolonne wird vorzugsweise so betrieben, dass die Leichtsieder als Kopfprodukt abgetrennt werden können.

Vorzugsweise wird der Verfahrenschritt e) in einer Destillationskolonne durchgeführt, die 30 bis 75 theoretische Trennstufen, bevorzugt 40 bis 65 und besonders bevorzugt 40 bis 55 theoretische Trennstufen aufweist. Vorzugsweise wird die Kolonne, in Abhängigkeit von der realisierten Stufenzahl, der Zusammensetzung des eingesetzten MTBE und der erforderlichen Reinheit an C₄- und C₅-Kohlenwasserstoffen, mit einem Rücklaufverhältnis zwischen 150 und 350, insbesondere zwischen 200 und 300 betrieben. Die Kolonne im optionalen Verfahrenschritt e) wird vorzugsweise mit einem Betriebsdruck von 0,2 bis 0,6 MPa_{(abs)}, bevorzugt von 0,3 bis 0,4 MPa_{(abs)} betrieben. Zur Beheizung der Kolonne kann z. B. Dampf eingesetzt werden. Die Kondensation kann, je nach gewähltem Betriebsdruck, gegen Kühlsohle, Kühlwasser oder Luft erfolgen. Der Kopfbrüden der Kolonne können vollständig oder nur teilweise kondensiert werden, so dass das Kopfprodukt VII entweder flüssig oder dampfförmig abgezogen werden kann. Das Kopfprodukt VII kann thermisch verwertet werden oder als Einsatzstoff einer Synthesegasanlage genutzt werden.

Werden in dem erfindungsgemäßen Verfahren Kolonnen eingesetzt, wie z. B. die in den Figuren Fig.1 bzw. Fig. 2 mit K1, K2 und K3 bezeichneten Kolonnen, so können diese mit Einbauten, die z. B. aus Böden, rotierenden Einbauten, regellosen Schüttungen und/oder geordneten Packungen sind, versehen sein.

Bei den Kolonnenböden können z. B. folgende Typen zum Einsatz kommen:
Böden mit Bohrungen oder Schlitzen in der Bodenplatte.
Böden mit Hälsen oder Kaminen, die von Glocken, Kappen oder Hauben überdeckt sind.
Böden mit Bohrungen in der Bodenplatte, die von beweglichen Ventilen überdeckt sind.
Böden mit Sonderkonstruktionen.

In Kolonnen mit rotierenden Einbauten kann der Rücklauf z. B. durch rotierende Trichter versprüht oder mit Hilfe eines Rotors als Film auf einer beheizten Rohrwand ausgebreitet werden.

In dem erfindungsgemäßen Verfahren können wie bereits gesagt Kolonnen eingesetzt werden, die regellose Schüttungen mit verschiedenen Füllkörpern aufweisen. Die Füllkörper können aus fast allen Werkstoffen, insbesondere z. B. aus Stahl, Edelstahl, Kupfer, Kohlenstoff, Steingut, Porzellan, Glas oder Kunststoffen bestehen und die verschiedensten Formen, insbesondere die Form von Kugeln, Ringen mit glatten oder profilierten Oberflächen, Ringen mit Innenstegen oder Wanddurchbrüchen, Drahtnetzringen, Sattelkörper und Spiralen aufweisen.

Packungen mit regelmäßiger/geordneter Geometrie können z. B. aus Blechen oder Geweben bestehen. Beispiele für solche Packungen sind Sulzer Gewebepackungen BX aus Metall oder Kunststoff, Sulzer Lamellenpackungen Mellapak aus Metallblech, Sulzer Hochleistungspackungen wie MellapakPlus, Strukturpackungen von Sulzer (Optiflow), Montz (BSH) und Kühni (Rombopak).

An Hand der Figuren Fig. 1 und 2 wird die vorliegende Erfindung nachfolgend näher erläutert.

Ein Blockschema einer Ausführungsform einer Anlage in der das erfindungsgemäße Verfahren durchgeführt werden kann, ist in Fig. 1 dargestellt. Der MTBE aufweisende Einsatzstrom I wird in der Kolonne K1 in ein Sumpfprodukt III, das die höher als MTBE siedenden Nebenkomponenten, wie beispielsweise Diisobuten und 2-Methoxybutan enthält, und in ein MTBE enthaltendes Kopfprodukt II getrennt. Das im Kopfprodukt II enthaltene MTBE wird im Reaktor R zum überwiegenden Teil in Isobuten und Methanol gespalten. Das im Reaktor R erhaltene Spaltprodukt IV wird in der Kolonne K2 in ein Isobuten aufweisendes Gemisch V, das Methanol und gegebenenfalls Dimethylether enthält, und in ein Sumpfprodukt VI, das nicht umgesetztes MTBE, Methanol und Nebenkomponenten enthält, fraktioniert.

Optional kann dem Einsatzstrom I ein Rückstrom VIII aus der Spaltung zugesetzt werden, in dem vornehmlich nicht umgesetztes MTBE enthalten ist. Ein solcher Rückstrom VIII kann z. B. das Sumpfprodukt VI der Kolonne K2 sein, wobei das darin enthaltene Methanol vor Rückführung durch einen oder mehrere Destillationsschritt teilweise oder vollständig abgetrennt werden sollte. Die Kolonne K2 kann optional als Reaktivdestillationskolonne ausgeführt werden.

Ein Blockschema einer weiteren Ausführungsform einer Anlage in der das erfindungsgemäße Verfahren durchgeführt werden kann, ist in Fig. 2 dargestellt. Die Ausführungsform von Fig. 2 unterscheidet sich von der von Fig. 1 darin, dass das Einsatz-MTBE Ia in einer Vorkolonne K3 in ein Leichtsieder, wie z. B. C₄- und C₅-Kohlenwasserstoffe, aufweisendes Kopfprodukt VII und einen von Leichtsiedern befreiten MTBE enthaltenden Strom I aufgetrennt wird, der dann in die Kolonne K1 eingespeist wird.

Die nachfolgenden Beispiele sollen die Erfindung erläutern.

### Beispiele

### Beispiel a: Herstellung eines Alumosilikatformkörpers

500 g Alumosilikatpulver (Hersteller: Grace Davison, Typ: Davicat O 701, formaler Al₂O₃-Gehalt: 13 Massen-%, formaler SiO₂-Gehalt: 76 Massen-%, formaler Na₂O-Gehalt: 0,1 Massen-%, Glühverlust bei 850 °C: ca. 11 %), 363 g Disperal-Gel (formaler Al₂O₃-Gehalt: 15,6 %), welches durch Einrühren von 197 g Disperal, einem Boehmit mit einem formalen Al₂O₃-Gehalt von 77 Massen-%, der Sasol Deutschland GmbH in 803 g einer 1,28 Massen-%igen, wässrigen Salpetersäure, anschließendes gründliches Rühren, bei dem das sich bildende Gel ständig geschert und so in einem fließfähigen Zustand gehalten wird, in einem abgedeckten Behälter für 3 h bei 60 °C, Abkühlen des Gels auf Raumtemperatur und Ersetzen von eventuell verdunstetem Wasser erhalten wird, und 370 g vollentsalztes Wasser (VE-Wasser) wurden zunächst gründlich miteinander in einem Intensiv-Mischer der Firma Eirich vermischt. Anschließend erfolgte eine Pelletierung in dem Intensiv-Mischer der Firma Eirich, bei der innerhalb von 30 bis 40 Minuten gleichmäßig rundliche Pellets mit einem Durchmesser von ca. 1 bis 3 mm erhalten wurden. Die feuchten Pellets wurden zunächst bei 120 °C im Luftstrom getrocknet und anschließend mit 2 K/min auf 550 °C aufgeheizt und bei dieser Temperatur für 10 h im Luftstrom kalziniert. Die so hergestellten Alumosilikatpellets enthielten formal 76 Massen-% Al₂O₃ und 24 Massen-% SiO₂. Weiterhin enthielt der hergestellte Katalysator 0,12 Massen-% Natriumverbindungen (berechnet als Natriumoxid). Die Zusammensetzung der Alumosilikatpellets wurde berechnet aus der Menge und der Zusammensetzung der Ausgangssubstanzen. Die Alumosilikatpellets wiesen ein Porenvolumen, bestimmt mit der oben beschriebenen Cyclohexanmethode, von 1,15 ml/g auf.

### Beispiel b: Herstellung eines geformten Katalysators

Aus VE-Wasser und Magnesiumnitrat-Hexahydrat wurde eine Imprägnierlösung mit einem Magnesiumgehalt von 4,7 Massen-% hergestellt. Der pH-Wert dieser Lösung betrug 5,1. Über eine Vakuumimprägnierung wurden eine ausgesiebte Fraktion des in Beispiel 1 hergestellten Alumosilikatträgers (Durchmesser: 1,0 mm bis 2,8 mm) mit der sauren Magnesiumnitratlösung getränkt. Dazu wurden die Pellets in ein Glasrohr gefüllt und dieses für etwa 30 min evakuiert (Wasserstrahlpumpenvakuum von ca. 25 hPa). Anschließend wurde die Imprägnierlösung von unten bis über den oberen Rand der Festkörperschüttung gesaugt. Nach einer Einwirkzeit von etwa 15 Minuten wurde die Lösung, die nicht von dem Träger aufgenommen worden war, abgelassen. Die feuchten Pellets wurden zunächst im Luftstrom bei 140 °C bis zur Gewichtskonstanz getrocknet und anschließend mit 3 K/min auf 450 °C aufgeheizt und bei dieser Temperatur für 12 h kalziniert. Der hergestellte Katalysator bestand formal aus 68 Massen-% Siliziumdioxid, aus 21 Massen-% Aluminiumoxid und aus 11 Massen-% Magnesiumoxid. Weiterhin enthielt der hergestellte Katalysator 0,11 Massen-% Natriumverbindungen (berechnet als Natriumoxid). Die Zusammensetzung des Katalysators wurde berechnet aus der Menge und der Zusammensetzung der Ausgangssubstanzen sowie der abgelaufenen Imprägnierlösung. Die Natriummengen waren Bestandteil des in Beispiel 1 eingesetzten Alumosilikats. Das Porenvolumen, bestimmt mit der oben beschrieben Cyclohexanmethode, betrug 1,1 ml/g.

Die nachfolgenden Beispielrechnungen wurden mit dem stationären Simulationsprogramm ASPEN Plus (Version 12.1 der Firma AspenTech) durchgeführt. Um transparente, reproduzierbare Daten zu erzeugen, wurden nur allgemein zugängliche Stoffdaten eingesetzt. Außerdem wurde bei allen Varianten auf den Einsatz einer Reaktivdestillation verzichtet. Durch diese Vereinfachungen ist es dem Fachmann leicht möglich, die Berechnungen nachzuvollziehen. Die eingesetzten Methoden besitzen zwar keine ausreichende Genauigkeit für die Auslegung technischer Anlagen, die qualitativen Unterschiede der Schaltungen werden aber korrekt erfasst. In allen gezeigten Varianten kann der MTBE-Umsatz durch Einsatz einer Reaktivdestillation erhöht werden.

In den Beispielen wurde die Property-Methode "UNIFAC-DMD" (s. J. Gmehling, J. Li, and M. Schiller, Ind. Eng. Chem. Res. 32, (1993), pp. 178-193) benutzt. Für den Reaktor R wird jeweils ein Reaktorvolumen von 100 1 modelliert, wobei eine Füllung mit einem Katalysator angenommen wird, der formal aus Magnesiumoxid, Aluminiumoxid und Siliziumoxid besteht und dessen Herstellung in den Beispielen a und b beschrieben wird.

Für die Reaktormodellierung wurde in den Rechnungen ein kinetisches Reaktormodell verwendet, das auf umfangreiche experimentelle Messdaten mit diesem Katalysator beruht. In den Beispielen werden daher jeweils auch die Reaktionstemperaturen genannt, die bei der Reaktormodellierung angenommen wurden. Da auch jeweils die Zusammensetzung der ein- und ausgehenden Ströme der Reaktionsstufe genannt werden, ist es dem Fachmann durch Nachstellung der Reaktoren mit fest vorgegebenen Umsätzen möglich, das Beispiel nachzurechnen, ohne die genauen Gleichungen für die Kinetik zu kennen. Der Reaktordruck beträgt in allen Beispielen 0,8 MPa_{(abs)}.

### Beispiel 1:

Das Beispiel 1 entspricht der in Fig. 2 dargestellten Variante ohne der optionalen Rückführung von Spaltprodukt zur Kolonne K1. Als Zulauf zu der MTBE-Spaltungsanlage gemäß Fig. 2 wurde ein MTBE-Strom (Ia) von 100 kg/h mit der in Tabelle 3 aufgeführten Zusammensetzung angenommen. Im Gegensatz zu handelsüblichen Kaftstoff-MTBE (s. Tabelle 1) war der Gehalt an 2-Methoxybutan erhöht. Dies ist typisch für MTBE aus MTBE-Syntheseanlagen, die mit einer MTBE-Spaltungsanlage gekoppelt sind. Einziger Auslass für 2-Methoxybutan in einer solchen Kopplungsanlage ist die Kolonne K1, bei der 2-Methoxybutan teilweise über Sumpf abgetrennt wird. Durch die Rückführung von Strömen aus der MTBE-Spaltung, die 2-Methoxybutan enthalten, in die MTBE-Synthese erhöht sich daher der Wert an 2-Methoxybutan im MTBE.

**Tabelle 3: Zusammensetzung des angenommenen MTBE-Eintrittstroms in die MTBE-Spaltungsanlage für Beispiel 1.**

| | Einsatz-MTBE (Ia) |
|---|---|
| Massenstrom [kg/h] | 100,00 |
| Massenanteile [kg/kg] | |
| Dimethylether | |
| Isobuten | |
| 1-Buten | 0,000080 |
| 2-Butene | 0,000920 |
| Pentane | 0,001500 |
| MTBE | 0,978150 |
| 2-Methoxybutan | 0,004500 |
| Methanol | 0,008500 |
| tert.-Butanol | 0,003000 |
| Wasser | 0,000050 |
| Diisobuten | 0,003300 |

Aus dem MTBE-Strom (Ia) werden zunächst in der Kolonne K3 die C₄- und C₅-Kohlenwasserstoffe bis auf einen Restgehalt von 50 Massen-ppm abgetrennt. Die Kolonne hat 50 theoretische Stufen und wird bei einem Rücklaufverhältnis von 210 und bei einem Druck von 0,5 MPa_{(abs)} betrieben. Der Zulauf des Roh-MTBE Ia erfolgt oberhalb von Stufe 20, gezählt von oben. Die Kopftemperatur beträgt 63,0 °C, die Sumpftemperatur beträgt 111,9 °C. Das Destillat dieser Kolonne (VII) hat einen Restgehalt von 10 Massen-% MTBE. Durch Erhöhung des Rücklaufverhältnisses und/oder der Trennstufenzahl ließe sich der MTBE-Gehalt weiter reduzieren. Tabelle 4 zeigt die Zusammensetzung des Destillatstroms (VII) und des Sumpfstroms der Kolonne K3.

**Tabelle 4: Zusammensetzung des Destillatstroms (VIII) und des Sumpfstroms der Kolonne K3 für Beispiel 1.**

| | Destillat K3 (VII) | Sumpfprodukt K3 |
|---|---|---|
| Massenstrom [kg/h] | 0,31 | 99,69 |
| Massenanteile [kg/kg] | | |
| Dimethylether | | |
| Isobuten | | |
| 1-Buten | 0,025943 | |
| 2-Butene | 0,298339 | |
| Pentane | 0,470265 | 0,000050 |
| MTBE | 0,100052 | 0,980866 |
| 2-Methoxybutan | 0,000023 | 0,004514 |
| Methanol | 0,089236 | 0,008250 |
| tert.-Butanol | | 0,003009 |
| Wasser | 0,016143 | |
| Diisobuten | | 0,003310 |

Der weitestgehend von Leichtsiedern befreite MTBE-Strom I (Sumpfprodukt der Kolonne K3) wird der Kolonne K1 zugeführt, in der vornehmlich Diisobuten und 2-Methoxybutan über Sumpf (III) abgetrennt werden. Die Kolonne hat 90 theoretische Stufen und wird bei einem Rücklaufverhältnis von 4,6 und bei einem Druck von 0,9 MPa_{(abs)} betrieben. Die Zugabe von Strom I erfolgt oberhalb Stufe 30, von oben gezählt. Die Kopftemperatur beträgt 141,3 °C, die Sumpftemperatur beträgt 145,8 °C. Als Kopfprodukt (II) wird eine gasförmige Fraktion erhalten, die über 98,5 Massen-% MTBE enthält. Der Gehalt an 2-Methoxybutan im Destillat wurde auf 2000 Massen-ppm eingestellt (s. Tabelle 5). Durch Erhöhung des Rücklaufverhältnisses und/oder der Trennleistung ließe sich der Gehalt an MTBE im Sumpfprodukt reduzieren.

**Tabelle 5: Zusammensetzung des Destillatstroms (II) und Sumpfstroms (III) der Kolonne K1 für Beispiel 1.**

| | Destillat K1 (II) | Sumpfprodukt K1 (III) |
|---|---|---|
| Massenstrom [kg/h] | 96,19 | 3,50 |
| Massenanteile [kg/kg] | | |
| Dimethylether | | |
| Isobuten | | |
| 1-Buten/2-Butene | | |
| Pentane | 0,000052 | |
| MTBE | 0,986281 | 0,832050 |
| 2-Methoxybutan | 0,002000 | 0,073603 |
| Methanol | 0,008550 | |
| tert.-Butanol | 0,003117 | 0,000062 |
| Wasser | | |
| Diisobuten | | 0,094286 |

Der Destillatstrom (II) der Kolonne K1 wird, nach weiterer Aufheizung auf Reaktionstemperatur, dem Reaktionsteil (R) zugeführt. Der Reaktor wird bei 300 °C und 0,8 MPa_{(abs)} gefahren. Bei diesen Reaktionsbedingungen ergibt sich ein MTBE-Umsatz von ca. 96 %, der Umsatz von 2-Methoxybutan beträgt ca. 22 %. Aufgrund der Begrenzung des Anteils an 2-Methoxybutan auf 2000 Massen-ppm im Reaktorzulauf ist aber trotz der Spaltung von 2-Methoxybutan zu 2-Buten die geforderte Spezifikation für lineare Butene im Isobutenprodukt nicht gefährdet. Die Zusammensetzung des Reaktoraustrags (IV) zeigt Tabelle 6.

**Tabelle 6: Zusammensetzung des Reaktoraustrags (IV) sowie des Destillatstroms (V) und des Sumpfstroms (VI) der Kolonne K2 für Beispiel 1.**

| | Reaktoraustrag (IV) | Destillat K2 (V) | Sumpfprodukt K2 (VI) |
|---|---|---|---|
| Massenstrom [kg/h] | 96,19 | 60,57 | 35,63 |
| Massenanteile [kg/kg] | | | |
| Dimethylether | 0,003430 | 0,005264 | 0,000311 |
| Isobuten | 0,604965 | 0,960218 | 0,001000 |
| 1-Buten /2-Butene | 0,000284 | 0,000449 | 0,000003 |
| Pentane | 0,000052 | 0,000076 | 0,000011 |
| MTBE | 0,037479 | | 0,101194 |
| 2-Methoxybutan | 0,001554 | | 0,004196 |
| Methanol | 0,348828 | 0,033809 | 0,884392 |
| tert.-Butanol | 0,000773 | | 0,002087 |
| Wasser | 0,001911 | 0,000183 | 0,004849 |
| Diisobuten | 0,000725 | | 0,001957 |

Der Reaktoraustrag (IV) wird teilweise kondensiert und zweiphasig der Kolonne K2 zugeführt. Die Kolonne hat 40 theoretische Stufen und wird bei einem Rücklaufverhältnis von 0.3 und bei einem Druck von 0,6 MPa_{(abs)} betrieben. Der Stoffzulauf erfolgt oberhalb Stufe 30, gezählt von oben. Die Kopftemperatur beträgt 48,4 °C, die Sumpftemperatur beträgt 114,8 °C. Das Sumpfprodukt besteht überwiegend aus nicht umgesetztem MTBE (ca. 10 Massen-%) und Methanol (ca. 88 Massen-%), siehe Tabelle 6. Dieses Sumpfprodukt kann in eine MTBE-Syntheseanlage zurückgefahren werden. Da in der Syntheseanlage üblicherweise kein Auslass für 2-Methoxybutan existiert, erhöht sich dadurch, wie in diesem Beispiel berücksichtigt, der 2-Methoxybutan-Gehalt im MTBE gegenüber MTBE, das in einer MTBE-Synthese ohne Kopplung mit einer MTBE-Spaltung produziert wird, siehe Tabellen 2 und 3.

Das Kopfprodukt (V) ist Isobuten mit einer Reinheit von größer 96 Massen-% Isobuten. Die in einer typischen Isobutenspezifikation geforderten Grenzen für lineare Butene (< 1000 Massen-ppm) und C₅-Kohlenwasserstoffe (< 1000 Massen-ppm) werden sicher eingehalten, siehe Tabelle 1 und 6. Durch eine Extraktion mit Wasser kann bei Bedarf das Methanol entfernt werden, das Restwasser und der Dimethylether können durch eine anschließende Destillation abgetrennt und das Isobuten auf eine Reinheit größer 99,9 Massen-% aufkonzentriert werden. Ein so weiter aufgereinigtes Isobuten genügt der in Tabelle 2 geforderten Spezifikation.

### Beispiel 2:

Das Beispiel 2 entspricht der in Fig. 2 dargestellten Variante mit Rückführung von Spaltprodukt zur Kolonne K1. Als Zulauf zu der MTBE-Spaltungsanlage wird gemäß Fig. 2 ein MTBE-Strom (Ia) von 100 kg/h mit der in Tabelle 7 aufgeführten Zusammensetzung angenommen (typisches Kraftstoff-MTBE, vergleiche mit Tabelle 2).

**Tabelle 7: Zusammensetzung des angenommen des MTBE-Eintrittstroms in die MTBE-Spaltungsanlage für Beispiel 2.**

| | Einsatz-MTBE (Ia) |
|---|---|
| Massenstrom [kg/h] | 100,00 |
| Massenanteile [kg/kg] | |
| Dimethylether | |
| Isobuten | |
| 1-Buten | 0,000080 |
| 2-Butene | 0,000920 |
| Pentane | 0,001500 |
| MTBE | 0,979650 |
| 2-Methoxybutan | 0,003000 |
| Methanol | 0,008500 |
| tert.-Butanol | 0,003000 |
| Wasser | 0,000050 |
| Diisobuten | 0,003300 |

Aus dem MTBE-Strom (Ia) werden zunächst wieder in der Kolonne K3 die Leichtsieder (C₄-und C₅-Kohlenwasserstoffe) bis auf einen Restgehalt von 50 Massen-ppm abgetrennt. Stufenzahl, Feedzugabestelle, Rücklaufverhältnis und Betriebsdruck der Kolonne K3 sind gegenüber Beispiel 1 unverändert. Die Kopftemperatur beträgt 63,0 °C, die Sumpftemperatur beträgt 111,8 °C. Das Destillat dieser Kolonne (VII) hat einen Restgehalt von 10 Massen-% MTBE. Durch Erhöhung des Rücklaufverhältnisses und/oder der Trennstufenzahl ließe sich der MTBE-Gehalt weiter reduzieren. Tabelle 8 zeigt die Zusammensetzung des Destillatstroms (VII) und des Sumpfstroms der Kolonne K3.

**Tabelle 8: Zusammensetzung des Destillatstroms (VIII) und des Sumpfstroms der Kolonne K3 für Beispiel 2.**

| | Destillat K3 (VII) | Sumpfprodukt K3 |
|---|---|---|
| Massenstrom [kg/h] | 0,31 | 99,69 |
| Massenanteile [kg/kg] | | |
| Dimethylether | | |
| Isobuten | | |
| 1-Buten | 0,025947 | |
| 2-Butene | 0,298386 | |
| Pentane | 0,470332 | 0,000050 |
| MTBE | 0,100001 | 0,982371 |
| 2-Methoxybutan | 0,000015 | 0,003009 |
| Methanol | 0,089175 | 0,008250 |
| tert.-Butanol | | 0,003009 |
| Wasser | 0,016145 | |
| Diisobuten | | 0,003310 |

Dem weitestgehend von Leichtsiedern befreiten MTBE wird ein Rückführstrom (VIII) aus der MTBE-Spaltung unter Erhalt des Stroms (I) zugemischt. Dabei handelt es sich um den Sumpfstrom (VI) der Kolonne K2, wobei angenommen wird, dass vor der Rückführung ein Großteil des Methanols und der Nebenkomponenten durch einen oder mehrere Destillationsschritte abgetrennt wurde. Die angenommene Zusammensetzung des Rückstroms (VIII) und des sich aus der Mischung ergebenen Feedstroms (I) zur Kolonne K1 ist in Tabelle 9 dargestellt.

**Tabelle 9: Zusammensetzung des Rückstroms aus der Spaltung (VIII) und des Feedstroms (I) zur Kolonne für Beispiel 2.**

| | Rückstrom (VIII) | Feed K1 (I) |
|---|---|---|
| Massenstrom [kg/h] | 22,99 | 122,69 |
| Massenanteile [kg/kg] | | |
| Dimethylether | | |
| Isobuten | | |
| 1-Buten | | |
| 2-Butene | | |
| Pentane | | 0,000041 |
| MTBE | 0,771297 | 0,942811 |
| 2-Methoxybutan | 0,011253 | 0,004554 |
| Methanol | 0,217450 | 0,047459 |
| tert.-Butanol | | 0,002445 |
| Wasser | | |
| Diisobuten | | 0,002690 |

Der durch Mischung aus dem Sumpfprodukt der Kolonne K3 und dem Rückführstrom (VIII) aus der MTBE-Spaltung entstandene Strom (I) wird der Kolonne K1 zugeführt, in der wieder vornehmlich Diisobuten und 2-Methoxybutan über Sumpf (III) abgetrennt werden. Stufenzahl, Stoffzulaufstelle und Betriebsdruck der Kolonne K1 sind gegenüber Beispiel 1 unverändert. Die Kolonne wird in Beispiel 2 bei einem Rücklaufverhältnis von 3,2 betrieben. Die Kopftemperatur beträgt 135,8 °C, die Sumpftemperatur beträgt 147,2 °C. Als Kopfprodukt (II) wird eine gasförmige Fraktion erhalten, die ca. 94,5 Massen-% MTBE enthält. Der Gehalt an 2-Methoxybutan im Destillat auf 2500 Massen-ppm eingestellt (s. Tabelle 10). Durch Erhöhung des Rücklaufverhältnisses und/oder der Trennleistung ließe sich der Gehalt an MTBE im Sumpfprodukt reduzieren.

**Tabelle 10: Zusammensetzung des Destillatstroms (II) und Sumpfstroms (III) der Kolonne K1 für Beispiel 2.**

| | Destillat K1 (II) | Sumpfprodukt K1 (III) |
|---|---|---|
| Massenstrom [kg/h] | 120,79 | 1,90 |
| Massenanteile [kg/kg] | | |
| Dimethylether | | |
| Isobuten | | |
| 1-Buten | | |
| 2-Butene | | |
| Pentane | 0,000041 | |
| MTBE | 0,946770 | 0,691130 |
| 2-Methoxybutan | 0,002500 | 0,135146 |
| Methanol | 0,048205 | |
| tert.-Butanol | 0,002483 | 0,000040 |
| Wasser | | |
| Diisobuten | | 0,173684 |

Der Destillatstrom der Kolonne K1 wird, nach weiterer Aufheizung auf Reaktionstemperatur, dem Reaktionsteil (R) zugeführt. Der Reaktor wird bei 265°C und 0,8 MPa_{(abs)} gefahren. Bei diesen Reaktionsbedingungen ergibt sich ein MTBE-Umsatz von ca. 85 %, der Umsatz von 2-Methoxybutan beträgt ca. 15 %. Aufgrund der Begrenzung von 2-Methoxybutan auf 2500 Massen-ppm im Reaktorzulauf ist aber trotz der Spaltung von 2-Methoxybutan zu linearen Butenen die geforderte Spezifikation für lineare Butene im Isobutenprodukt nicht gefährdet. Die Zusammensetzung des Reaktoraustrags (IV) zeigt Tabelle 11.

**Tabelle 11: Zusammensetzung des Reaktoraustrags (IV) sowie des Destillatstroms (V) und des Sumpfstroms (VI) der Kolonne K2 für Beispiel 2.**

| | Reaktoraustrag (IV) | Destillat K2 (V) | Sumpfprodukt K2 (VI) |
|---|---|---|---|
| Massenstrom [kg/h] | 120,79 | 64,09 | 56,70 |
| Massenanteile [kg/kg] | | | |
| Dimethylether | 0,002742 | 0,005144 | 0,000026 |
| Isobuten | 0,510121 | 0,960544 | 0,001000 |
| 1-Buten /2-Butene | 0,000241 | 0,000451 | 0,000003 |
| Pentane | 0,000041 | 0,000063 | 0,000017 |
| MTBE | 0,146749 | | 0,312622 |
| 2-Methoxybutan | 0,002122 | | 0,004521 |
| Methanol | 0,335334 | 0,033554 | 0,676443 |
| tert.-Butanol | 0,000546 | | 0,001164 |
| Wasser | 0,001543 | 0,000243 | 0,003012 |
| Diisobuten | 0,000560 | | 0,001193 |

Der Reaktoraustrag (IV) wird teilweise kondensiert und zweiphasig der Kolonne K2 zugeführt. Stufenzahl, Stoffzulaufstelle, Rücklaufverhältnis und Betriebsdruck der Kolonne K2 sind gegenüber Beispiel 1 unverändert. Die Kopftemperatur beträgt 48,3 °C, die Sumpftemperatur beträgt 111,4 °C Das Sumpfprodukt besteht wieder überwiegend aus nicht umgesetztem MTBE (ca. 31 Massen-%) und Methanol (ca. 67 Massen-%), siehe Tabelle 11. Aufgrund des gegenüber Beispiel 1 deutlich höheren Rest-MTBE-Gehaltes ist es in diesem Beispiel lohnenswert, den Großteil des Methanols und weitere Nebenkomponenten durch einen oder mehren Destillationsschritte abzutrennen und den so aufgereinigten Strom zurück vor die Kolonne K1 zu fahren. So kann die Gesamtausbeute an Isobuten, bezogen auf den MTBE-Einsatz, deutlich erhöht werden.

Das Kopfprodukt (V) ist Isobuten mit einer Reinheit von größer 96 Massen-% Isobuten. Die in einer typischen Isobutenspezifikation geforderten Grenzen für lineare Butene (< 1000 Massen-ppm) und C₅-Kohlenwasserstoffe (< 1000 Massen-ppm) werden sicher eingehalten, siehe Tabelle 1 und 11. Durch eine Extraktion mit Wasser kann bei Bedarf das Methanol entfernt werden, das Restwasser und der Dimethylether können durch eine anschließende Destillation abgetrennt und das Isobuten auf eine Reinheit größer 99,9 Massen-% aufkonzentriert werden. Ein so weiter aufgereinigtes Isobuten genügt der in Tabelle 1 geforderten Spezifikation.

## Patentansprüche

1. Verfahren zur Spaltung von Methyl-tert.-butylether (MTBE), das zumindest die Schritte
a) destillative Trennung eines MTBE aufweisenden Stroms I in einen MTBE aufweisenden Kopfstrom II, der eine 2-Metoxybutan-Konzentration von mehr als 1000 Massen-ppm bis zu 2500 Massen-ppm, jeweils bezogen auf das MTBE, aufweist und einen Sumpfstrom III, der höher als MTBE siedende Verbindungen aufweist, und
b) Spaltung des im Kopfstrom II enthaltenen MTBE bei MTBE-Umsätzen von 85 bis 95 % an einem Katalysator unter Erhalt eines Spaltproduktes IV aufweist,
**dadurch gekennzeichnet,**
**dass** der Strom I einen Anteil an 2-Methoxybutan (MSBE) von größer 1000 Massen-ppm in Bezug auf MTBE aufweist und dass die destillative Trennung in Schritt a) und die Spaltung in Schritt b) so durchgeführt wird, dass das erhaltene Spaltprodukt IV eine Konzentration von weniger als 1000 Massen-ppm an linearen Butenen bezogen auf die C₄-Olefinfraktion aufweist,
wobei ein Katalysator eingesetzt wird, der einen Anteil an Magnesiumoxid von 0,5 bis 20 Massen-%, einen Anteil an Aluminiumoxid von 4 bis 30 Massen-% und einen Anteil an Siliziumdioxid von 60 bis 95 Massen-% aufweist.

2. Verfahren nach Anspruch 1,
**dadurch gekennzeichnet,**
**dass** als Strom I technisches MTBE eingesetzt wird, enthaltend 1-Buten, 2-Butene, Pentane, MTBE, 2-Methoxybutan, Methanol, tert-Butanol, Wasser, Diisobuten.

3. Verfahren nach zumindest einem der Ansprüche 1 bis 2,
**dadurch gekennzeichnet,**
**dass** das Spaltprodukt IV in einem weiteren Verfahrensschritt c) destillativ in einen Isobuten aufweisenden Kopfstrom V und einen nicht umgesetztes MTBE aufweisenden Sumpfstrom VI getrennt wird.

4. Verfahren nach Anspruch 3,
**dadurch gekennzeichnet,**
**dass** aus dem in Verfahrensschritt c) erhaltenen Isobuten aufweisenden Kopfstrom V in einem weiteren Verfahrensschritt d) Methanol durch Extraktion und aus dem extrahierten Isobuten Dimethylether und Wasser destillativ abgetrennt werden.

5. Verfahren nach zumindest einem der Ansprüche 1 bis 4,
**dadurch gekennzeichnet,**
**dass** als Strom I ein Gemisch aus technischem MTBE und einem destillativ abgetrennten Teilstrom des Spaltproduktes IV eingesetzt wird.

6. Verfahren nach zumindest einem der Ansprüche 1 bis 5,
**dadurch gekennzeichnet,**
**dass** als Strom I ein MTBE aufweisender Strom eingesetzt wird, der ganz oder teilweise durch Abtrennen von Leichtsiedern aus einem MTBE aufweisenden Strom Ia erhalten wird.

## Claims

1. Process for the dissociation of methyl tert-butyl ether (MTBE), which comprises at least the steps
a) separation by distillation of an MTBE-containing stream I into an MTBE-containing overhead stream II which has a 2-methoxybutane concentration of more than 1000 ppm by mass up to 2500 ppm by mass, in each case based on the MTBE, and a bottom stream III which comprises compounds having boiling points higher than that of MTBE and
b) dissociation of the MTBE present in the overhead stream II at MTBE conversions of from 85 to 95% over a catalyst to give a dissociation product IV,
**characterized in that**
the stream I has a proportion of 2-methoxybutane (MSBE) of greater than 1000 ppm by mass based on MTBE and **in that** the separation by distillation in step a) and the dissociation in step b) is carried out so that the dissociation product IV obtained has a concentration of less than 1000 ppm by mass of linear butenes, based on the C₄-olefin fraction, where a catalyst which has a proportion of magnesium oxide of from 0.5 to 20% by mass, a proportion of aluminium oxide of from 4 to 30% by mass and a proportion of silicon dioxide of from 60 to 95% by mass is used.

2. Process according to Claim 1,
**characterized in that**
industrial MTBE containing 1-butene, 2-butenes, pentanes, MTBE, 2-methoxybutar methanol, tert-butanol, water, diisobutene is used as stream I.

3. Process according to at least one of Claims 1 to 2,
**characterized in that**
the dissociation product IV is separated by distillation in a further process step c) into an isobutene-containing overhead stream V and a bottom stream VI comprising unreacted MTBE.

4. Process according to Claim 3,
**characterized in that**,
in a further process step d), methanol is separated off from the isobutene-containing overhead stream V obtained in process step c) by extraction and dimethyl ether and water are separated off by distillation from the extracted isobutene.

5. Process according to at least one of Claims 1 to 4,
**characterized in that**
a mixture of industrial MTBE and a substream separated off from the dissociation product IV by distillation is used as stream I.

6. Process according to at least one of Claims 1 to 5,
**characterized in that**
an MTBE-containing stream which is entirely or partly obtained by removing low boilers from an MTBE-containing stream Ia is used as stream I.

## Revendications

1. Procédé pour la dissociation de méthyl-tert-butyléther (MTBE), qui présente au moins les étapes de
a) séparation par distillation d'un flux I présentant du MTBE en un flux de tête II présentant du MTBE, qui présente une concentration en 2-métoxybutane supérieure à 1000 ppm en masse jusqu'à 2500 ppm en masse, à chaque fois par rapport au MTBE, et en un flux de fond III qui présente les composés à point d'ébullition supérieur à celui du MTBE et
b) dissociation du MTBE contenu dans le flux de tête II à des conversions de MTBE de 85 à 95% sur un catalyseur avec obtention d'un produit de dissociation IV,
**caractérisé en ce que** le flux I présente une proportion de 2-méthoxybutane (MSBE) supérieure à 1000 ppm en masse par rapport au MTBE et **en ce que** la séparation par distillation dans l'étape a) et la dissociation dans l'étape b) sont réalisées de manière telle que le produit de dissociation IV obtenu présente une concentration inférieure à 1000 ppm en masse en butènes linéaires par rapport à la fraction oléfinique en C₄,
un catalyseur, présentant une proportion d'oxyde de magnésium de 0,5 à 20% en masse, une proportion d'oxyde d'aluminium de 4 à 30% en masse et une proportion de dioxyde de silicium de 60 à 95% en masse, étant utilisé.

2. Procédé selon la revendication 1, **caractérisé en ce qu'**on utilise, comme flux I, du MTBE de qualité technique, contenant du 1-butène, des 2-butènes, des pentanes, du MTBE, du 2-méthoxybutane, du méthanol, du tert-butanol, de l'eau, des diisobutènes.

3. Procédé selon au moins l'une quelconque des revendications 1 à 2, **caractérisé en ce que** le produit de dissociation IV est séparé par distillation dans une étape de procédé c) en un flux de tête V présentant de l'isobutène et en un flux de fond VI présentant du MTBE non transformé.

4. Procédé selon la revendication 3, **caractérisé en ce qu'**on sépare du flux de tête V obtenu dans l'étape de procédé c), présentant de l'isobutène, dans une autre étape de procédé d), du méthanol par extraction et, de l'isobutène extrait, du diméthyléther et de l'eau par distillation.

5. Procédé selon au moins l'une quelconque des revendications 1 à 4, **caractérisé en ce qu'**on utilise, comme flux I, un mélange de MTBE de qualité technique et d'un flux partiel séparé par distillation du produit de dissociation IV.

6. Procédé selon au moins l'une quelconque des revendications 1 à 5, **caractérisé en ce qu'**on utilise, comme flux I, un flux présentant du MTBE, qui est obtenu totalement ou partiellement par séparation de fractions à bas point d'ébullition d'un flux la présentant du MTBE.
